# EUROPÄISCHE PATENTSCHRIFT

(11) **EP 0 916 679 B1**
(45) Veröffentlichungstag und Bekanntmachung des Hinweises auf die Patenterteilung: **20.11.2002**
(21) Anmeldenummer: 98120898.6
(22) Anmeldetag: 04.11.1998
(51) Int. Cl.: C07K 5/06, A61K 38/05

(54) **Phosphonsäure-substituierte Benzazepinon-N-essigsäurederivate sowie Verfahren zu ihrer Herstellung und diese Verbindungen enthaltende Arzneimittel**
Benzazepinone-N-acetic acid derivatives having a phosphonic acid group, process for their preparation and medicaments containing these compounds
Dérivés d'acides benzazepinone-N-acétiques ayant un groupe phosphonique, leur procédé de préparation et médicaments contenant lesdits composés

(30) Priorität: 12.11.1997 DE 19750002
(43) Veröffentlichungstag der Anmeldung: 19.05.1999
(73) Patentinhaber: Solvay Pharmaceuticals GmbH, 30173 Hannover (DE)
(72) Erfinder: Waldeck, Harald, Dr., 30916 Isernhagen HB (DE); Meil, Jörg, Dr., 30890 Barsinghausen (DE); Thormählen, Dirk, Dr., 31039 Rheden (DE); Wurl, Michael, Dr., 30826 Schloss Ricklingen (DE)
(74) Vertreter: Gosmann, Martin, Dr.

(56) Entgegenhaltungen:
- EP-A- 0 733 642
- EP-A- 0 743 319
- EP-A- 0 747 392

## Beschreibung

Die vorliegende Erfindung betrifft neue Benzazepinon-N-essigsäure-Derivate, welche in 3-Stellung durch einen in 1-Stellung einen Methylphosphonsäurerest tragenden Cyclopentylcarbonylamino-Rest substituiert sind, und deren Salze und biolabile Ester sowie diese Verbindungen enthaltende pharmazeutische Zubereitungen und Verfahren zur Herstellung dieser Verbindungen.

Aus der europäischen Patentanmeldung, Veröffentlichungsnummer 0 733 642, sind Benzazepin-, Benzoxazepin- und Benzodiazepin-N-essigsäurederivate bekannt, welche eine Hemmwirkung auf die neutrale Endopeptidase (= NEP) ausüben.

Aufgabe der Erfindung ist es, neue NEP-inhibitorisch wirksame pharmazeutische Wirkstoffe mit einem für die Behandlung von Herzinsuffizienz und Bluthochdruck günstigen Wirkprofil zu entwickeln.

Es wurde nun gefunden, daß die erfindungsgemäßen neuen Benzazepinon-N-essigsäure-Derivate, welche in 3-Stellung des Benzazepinon-Gerüstes durch einen in 1-Stellung einen Methylphosphonsäurerest tragenden Cyclopentylcarbonylamino-Rest substituiert sind, wertvolle herzwirksame pharmakologische Eigenschaften besitzen und sich durch ein zur Behandlung von kardiovaskulären Erkrankungen, insbesondere Herzinsuffizienz, günstiges Wirkprofil auszeichnen, welches durch eine Kombination von einer ausgeprägten Hemmwirkung auf die neutrale Endopeptidase mit einer Hemmwirkung auf das Endothelin Konvertierende Enzym (= ECE) und eine gute Verträglichkeit gekennzeichnet ist.

Gegenstand der Erfindung sind neue Verbindungen der allgemeinen Formel I 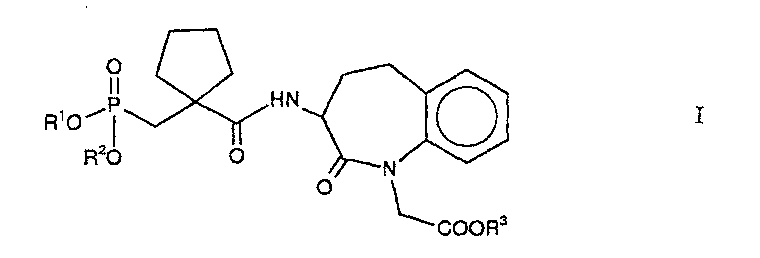 worin
- R¹: Wasserstoff oder eine einen biolabilen Phosphonsäureester bildende Gruppe bedeutet,
- R²: Wasserstoff oder eine einen biolabilen Phosphonsäureester bildende Gruppe bedeutet und
- R³: Wasserstoff oder eine einen biolabilen Carbonsäureester bildende Gruppe bedeutet
und physiologisch verträgliche Salze von Säuren der Formel I sowie Verfahren zur Herstellung dieser Verbindungen und diese Verbindungen enthaltende Arzneimittel.

Die Verbindungen der Formel I stellen gegebenenfalls durch biolabile Ester bildende Gruppen veresterte, Carbonsäure- und Phosphonsäuregruppen enthaltende Säurederivate dar. Die biolabilen Ester der Formel I stellen Prodrugs der freien Säuren dar. Je nach Applikationsform sind die biolabilen Ester oder die Säuren bevorzugt, wobei letztere insbesondere für die i.v.-Applikation geeignet sind.

Als biolabile Phosphonsäureester bildende Gruppen R¹ und R² sind Gruppen geeignet, welche unter physiologischen Bedingungen in vivo unter Freisetzung der jeweiligen Phosphonsäurefunktion abgespalten werden können. Beispielsweise eignen sich hierfür niedere Alkylgruppen, gegebenenfalls an der Oxymethylgruppe durch niederes Alkyl substituierte C₂-C₆-Alkanoyloxymethylgruppen oder Phenyl- oder Phenylniederalkylgrupen, deren Phenylring gegebenenfalls ein- oder mehrfach durch niederes Alkyl, niederes Alkoxy oder durch eine an zwei benachbarte Kohlenstoffatome gebundene niedere Alkylenkette substituiert ist. Sofern die einen biolabilen Ester bildende Gruppe R¹ und/oder R² niederes Alkyl bedeutet oder enthält, kann dieses verzweigt oder unverzweigt sein und 1 bis 4 Kohlenstoffatome enthalten. Sofern R¹ und/oder R² eine gegebenenfalls substituierte Alkanoyloxymethylgruppe darstellen, kann diese eine vorzugsweise verzweigte Alkanoyloxygruppe mit 2 bis 6, vorzugsweise 3 bis 5 Kohlenstoffatomen enthalten und kann beispielsweise für einen Pivaloyloxymethylrest (= tert.-Butylcarbonyloxymethylrest) stehen. Sofern R¹ und/oder R² eine gegebenenfalls substituierte Phenylniederalkylgruppe darstellen, kann diese eine Alkylenkette mit 1 bis 3, vorzugsweise 1, Kohlenstoffatomen enthalten. Sofern der Phenylring durch eine niedere Alkylenkette substituiert ist, kann diese 3 bis 4, insbesondere 3, Kohlenstoffatome enthalten und der substituierte Phenylring stellt insbesondere Indanyl dar.

Als biolabile Carbonsäureester bildende Gruppen R³ sind Gruppen geeignet, welche unter physiologischen Bedingungen in vivo unter Freisetzung der Carbonsäure gespalten werden können. Beispielsweise eignen sich hierfür niedere Alkylgruppen, gegebenenfalls im Phenylring ein- oder mehrfach durch niederes Alkyl oder niederes Alkoxy oder durch eine an zwei benachbarte Kohlenstoffatome gebundene niedere Alkylenkette substituierte Phenyl- oder Phenylniederalkylgruppen, im Dioxolanring gegebenenfalls durch niederes Alkyl substituierte Dioxolanylmethylgruppen oder gegebenenfalls an der Oxymethylgruppe durch niederes Alkyl substituierte C₂-C₆-Alkanoyloxymethylgruppen. Sofern die einen biolabilen Ester bildende Gruppe R³ niederes Alkyl bedeutet oder enthält, kann dieses verzweigt oder unverzweigt sein und 1 bis 4 Kohlenstoffatome enthalten. Sofern die einen biolabilen Ester bildende Gruppe eine gegebenenfalls substituierte Phenylniederalkylgruppe darstellt, kann diese eine Alkylenkette mit 1 bis 3, vorzugsweise 1, Kohlenstoffatomen enthalten und steht vorzugsweise für Benzyl. Sofern der Phenylring durch eine niedere Alkylenkette substituiert ist, kann diese 3 bis 4, vorzugsweise 3 Kohlenstoffatome enthalten. Sofern R³ eine gegebenenfalls substituierte Alkanoyloxymethylgruppe darstellt, kann diese eine vorzugsweise verzweigte Alkanoyloxygruppe mit 2 bis 6, vorzugsweise 3 bis 5, Kohlenstoffatomen enthalten und kann beispielsweise einen Pivaloyloxymethylrest darstellen.

Erfindungsgemäß werden die neuen Verbindungen der Formel I und deren Salze erhalten, indem man in an sich bekannter Weise
a) zur Herstellung von Verbindungen der allgemeinen Formel IV 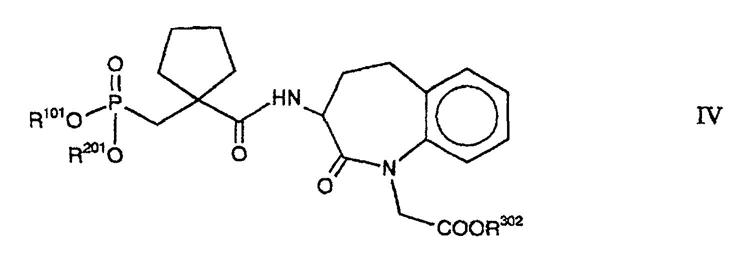 worin R¹⁰¹ und R²⁰¹ jeweils unabhängig voneinander Wasserstoff oder eine Phosphonsäureschutzgruppe bedeuten und R³⁰² eine Carbonsäureschutzgruppe bedeutet, Verbindungen der allgemeinen Formel II 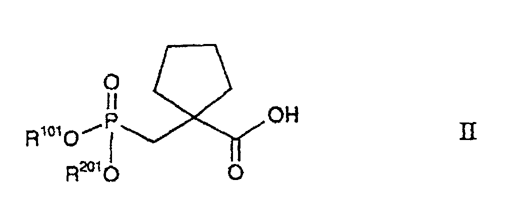 worin R¹⁰¹ und R²⁰¹ obige Bedeutungen besitzen, mit Verbindungen der allgemeinen Formel III 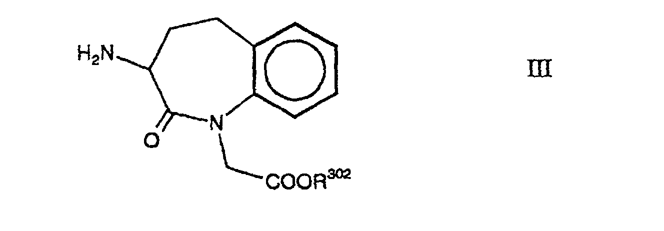 worin R³⁰² obige Bedeutung besitzt, umsetzt und, falls R¹⁰¹ und/oder R²⁰¹ Wasserstoff bedeuten, die freie(n) Phosphonsäurefunktion(en) gewünschtenfalls durch Veresterung mit einer Verbindung der allgemeinen Formel Va und/oder Vb,

   R¹¹⁰-Y (Va) R²¹⁰-Y (Vb)

   worin R¹¹⁰ und R²¹⁰ jeweils eine einen biolabilen Phosphonsäureester bildende Gruppe bedeuten und Y Hydroxy oder eine abspaltbare Fluchtgruppe bedeutet, in eine biolabile Phosphonsäureestergruppe überführt und
b) sofern in den Verbindungen der Formel IV die Schutzgruppen R¹⁰¹, R²⁰¹ und/oder R³⁰² keine gewünschten, einen biolabilen Ester bildende Gruppen darstellen, diese gleichzeitig oder einzeln in beliebiger Reihenfolge nacheinander abspaltet
   und gewünschtenfalls die jeweils freiwerdenden Säurefunktionen in biolabile Estergruppen überführt, indem man freie Phosphonsäurefunktionen mit einer Verbindung der Formel Va oder Vb verestert und/oder die freie Carbonsäurefunktion mit einer Verbindung der allgemeinen Formel Vc

   R³¹⁰-Y (Vc)

   worin R³¹⁰ eine einen biolabilen Carbonsäureester bildende Gruppe bedeutet und Y obige Bedeutung besitzt, verestert,
und gewünschtenfalls Säuren der Formel I in ihre physiologisch verträglichen Salze überführt oder Salze der Säuren der Formel I in die freien Verbindungen überführt.

Als physiologisch verträgliche Salze von Säuren der Formel I kommen jeweils deren Alkalimetall-, Erdalkalimetalloder Ammoniumsalze in Frage, beispielsweise deren Natrium-, Kalium- oder Calciumsalze oder Salze mit physiologisch verträglichen, pharmakologisch neutralen organischen Aminen wie beispielsweise Diethylamin, tert.-Butylamin oder Phenylniederalkylaminen wie α-Methylbenzylamin.

Als Phosphonsäureschutzgruppen R¹⁰¹ und R²⁰¹ können an sich zum Schutz von Phosphonsäurefunktionen übliche Schutzgruppen gewählt werden, die anschließend nach an sich bekannten Methoden wieder abgespalten werden. Als Carbonsäureschutzgruppen R³⁰² können an sich zum Schutz von Carbonsäurefunktionen übliche Schutzgruppen gewählt werden, die anschließend nach an sich bekannten Methoden wieder abgespalten werden können. Geeignete Schutzgruppen für Carbonsäuren sind beispielsweise bekannt aus McOmie, "Protective Groups in Organic Chemistry", Plenum Press und Green, Wuts, "Protective Groups in Organic Synthesis", Wiley Interscience Publication. Geeignete Schutzgruppen für Phosphonsäuren sind beispielsweise bekannt aus Houben-Weyl "Methoden der Organischen Chemie" G. Thieme Verlag Stuttgart, New York 1982, Seiten 313-341 sowie aus M. Kluba, A. Zwierak "Synthesis" 1978, Seiten 134-137 und aus McOmie, "Protective Groups in Organic Chemistry", Plenum Press. Als Säureschutzgruppen können auch einen biolabilen Ester bildende Gruppen eingesetzt werden. Die bei der Umsetzung von Verbindungen der Formel II mit Verbindungen der Formel III erhaltenen Verbindungen der Formel IV stellen in diesen Fällen bereits erfindungsgemäße Ester der Formel I dar.

Erfindungsgemäß eignen sich als Phosphonsäureschutzgruppen R¹⁰¹ und R²⁰¹ solche Gruppen, welche sich nach geeigneten Methoden unabhängig voneinander und unabhängig von einer im Molekül etwa noch vorhandenen Carbonsäureschutzgruppe R³⁰² selektiv abspalten oder selektiv einführen lassen. In der Regel können Phosphonsäureschutzgruppen leicht mittels Trimethylsilylbromid selektiv in Gegenwart von Carbonsäureschutzgruppen abgespalten werden. Als Beispiele für unter unterschiedlichen Bedingungen abspaltbare Phosphonsäureschutzgruppen, welche auch biolabile Phosphonsäureester bildende Gruppen darstellen können, seien nachfolgend genannt: unverzweigte niedere Alkylgruppen wie Ethyl, welche beispielsweise durch Säuren wie Trifluoressigsäure leicht abgespalten werden können, wobei, sofern beide Phosphonsäurefunktionen mit niederen unverzweigten Alkylgruppen verestert sind, unter basischen Bedingungen nur eine dieser Alkylgruppen abgespalten werden kann. Verzweigte niedere Alkylgruppen wie tert.-Butyl, welche unter sauren Bedingungen, beispielsweise unter der Einwirkung von Trifluoressigsäure, leicht abgespalten werden können. Gegebenenfalls im Phenylring substituierte Phenylmethylgruppen wie Benzyl, welche leicht durch Hydrogenolyse abgespalten werden können. Alkanoyloxymethylgruppen wie Pivaloyloxymethyl, welche beispielsweise durch Säuren wie Trifluoressigsäure leicht abgespalten werden können. Im Phenylring ein- oder mehrfach durch niederes Alkoxy substituierte Phenylmethylgruppen wie p-Methoxybenzyl, welche unter oxidativen Bedingungen, beispielsweise unter der Einwirkung von 2,3-Dichlor-5,6-dicyan-1,4-benzochinon (= DDQ) oder Cerammoniumnitrit (= CAN), relativ leicht abgespalten werden können.

Als Carbonsäureschutzgruppen R³⁰² eignen sich solche Gruppen, welche sich unabhängig von etwa noch im Molekül vorhandenen Phosphonsäureschutzgruppen selektiv abspalten oder selektiv einführen lassen. Als Beispiele für unter unterschiedlichen Bedingungen abspaltbare Carbonsäureschutzgruppen, welche auch biolabile Carbonsäureester bildende Gruppen darstellen können, seien genannt: unverzweigte niedere Alkylgruppen wie Ethyl, welche unter basischen Bedingungen verhältnismäßig leicht abgespalten werden können. Verzweigte niedere Alkylgruppen wie tert.-Butyl, welche leicht durch Säuren wie Trifluoressigsäure abgespalten werden können. Gegebenenfalls im Phenylring substituierte Phenylmethylgruppen wie Benzyl, welche leicht hydrogenolytisch oder auch unter basischen Bedingungen abgespalten werden können. Im Phenylring ein- oder mehrfach durch niederes Alkoxy substituierte Phenylmethylgruppen wie p-Methoxybenzyl, welche unter oxidativen Bedingungen, beispielsweise unter der Einwirkung von DDQ oder CAN, relativ leicht abgespalten werden.

Die Verbindungen der Formel I enthalten ein chirales Kohlenstoffatom, nämlich das die Amidseitenkette tragende Kohlenstoffatom in 3-Stellung des Benzazepingerüstes. Die Verbindungen können somit in zwei optisch aktiven stereoisomeren Formen oder als Racemat vorliegen. Die vorliegende Erfindung umfaßt sowohl die racemischen Gemische wie auch die isomerenreinen Verbindungen der Formel I. Sofern in Verbindungen der Formel I R¹ und R² nicht für Wasserstoff stehen und jeweils unterschiedliche Bedeutungen besitzen, kann auch das Phosphoratom der Phosphonsäuregruppe chiral sein. Auch die durch chirale Phosphoratome entstehenden Isomerengemische und isomerenreinen Verbindungen der Formel I sind Gegenstand der Erfindung.

Die Umsetzung der Säuren der Formel II mit den Aminen der Formel III zu den Amiden der Formel IV kann nach an sich zur Bildung von Amidgruppierungen durch Aminoacylierung üblichen Methoden ausgeführt werden. Als Acylierungsmittel können die Carbonsäuren der Formel II oder deren reaktionsfähige Derivate eingesetzt werden. Als reaktionsfähige Derivate kommen insbesondere gemischte Säureanhydride und Säurehalogenide in Frage. So können beispielsweise Säurechloride oder Säurebromide der Säuren der Formel II oder gemischte Ester der Säuren der Formel II mit organischen Sulfonsäuren, beispielsweise mit gegebenenfalls durch Halogen substituierten Niederalkansulfonsäuren wie Methansulfonsäure oder Trifluormethansulfonsäure oder mit aromatischen Sulfonsäuren wie z.B. Benzolsulfonsäuren oder mit durch niederes Alkyl oder Halogen substituierten Benzolsulfonsäuren, z. B. Toluolsulfonsäuren oder Brombenzolsulfonsäuren, eingesetzt werden. Die Acylierung kann in einem unter den Reaktionsbedingungen inerten organischen Lösungsmittel bei Temperaturen zwischen -20 °C und Raumtemperatur erfolgen. Als Lösungsmittel eignen sich halogenierte Kohlenwasserstoffe wie Dichlormethan oder aromatische Kohlenwasserstoffe wie Benzol oder Toluol oder cyclische Ether wie Tetrahydrofuran (= THF) oder Dioxan oder Gemische dieser Lösungsmittel.

Die Acylierung kann zweckmäßig, insbesondere wenn als Acylierungsmittel ein gemischtes Anhydrid der Säuren der Formel II mit einer Sulfonsäure verwendet wird, in Gegenwart eines säurebindenden Reagenzes durchgeführt werden. Als säurebindende Mittel eignen sich beispielsweise in dem Reaktionsgemisch lösliche organische Basen wie tertiäre Stickstoffbasen, beispielsweise tert.-Niederalkylamine und Pyridine wie z. B. Triethylamin, Tripropylamin, N-Methylmorpholin, Pyridin, 4-Dimethylaminopyridin, 4-Diethylaminopyridin oder 4-Pyrrolidinopyridin. Im Überschuß eingesetzte organische Basen können gleichzeitig auch als Lösungsmittel dienen.

Falls als Acylierungsmittel die Säuren der Formel II selbst eingesetzt werden, kann die Umsetzung der Aminoverbindungen der Formel III mit den Carbonäuren der Formel II zweckmäßig auch in Gegenwart eines aus der Peptidchemie als zur Amidbildung geeignet bekannten Kopplungsreagenzes durchgeführt werden. Als Beispiel von Kopplungsreagenzien, welche die Amidbildung mit den freien Säuren dadurch fördern, daß sie mit der Säure in situ unter Bildung eines reaktionsfähigen Säurederivates reagieren, seien insbesondere genannt: Alkylcarbodiimide, z. B. Cycloalkylcarbodiimide wie Dicyclohexylcarbodiimid oder N-(3-Dimethylaminopropyl)-N'-ethylcarbodiimid, Carbonyldiimidazol und N-Niederalkyl-2-Halogenpyridiniumsalze, insbesondere Halogenide oder Toluolsulfonate. Die Umsetzung in Gegenwart eines Kopplungsreagenzes kann zweckmäßig bei Temperaturen von -30° bis +50 °C in Lösungsmittein wie halogenierten Kohlenwasserstoffen und/oder aromatischen Lösungsmitteln und gegebenenfalls in Gegenwart eines vorstehend beschriebenen säurebindenden Amins durchgeführt werden.

Aus den durch Umsetzung der Verbindungen der Formel II mit den Verbindungen der Formel III erhaltenen Verbindungen der Formel IV können die Schutzgruppen R¹⁰¹, R²⁰¹ und R³⁰², sofern sie keine gewünschten, einen biolabilen Ester bildende Gruppen darstellen, auf an sich bekannte Weise abgespalten werden.

Sofern Verbindungen der Formel I hergestellt werden sollen, in denen R¹, R² und R³ für identische, einen biolabilen Ester bildende Gruppen stehen, werden zweckmäßigerweise bereits in den Ausgangsverbindungen der Formel II und in den Ausgangsverbindungen der Formel III identische Schutzgruppen gewählt. Zweckmäßigerweise können hierbei Schutzgruppen gewählt werden, welche gleichzeitig biolabile Ester bildende Gruppen darstellen. Sofern freie Säuren der Formel I hergestellt werden sollen, worin R¹, R² und R³ jeweils für Wasserstoff stehen, können als Schutzgruppen R¹⁰¹, R²⁰¹ und R³⁰² jeweils unter den gleichen Bedingungen, vorzugsweise unter hydrogenolytischen Bedingungen abspaltbare Gruppen gewählt werden. Beispielsweise können für R¹⁰¹, R²⁰¹ und R³⁰² jeweils Benzylgruppen gewählt werden, welche unter den Bedingungen einer katalytischen Hydrierung gleichzeitig zu den freien Säuregruppen gespalten werden. Als Katalysatoren für die katalytische Hydrierung können beispielsweise Edelmetallkatalysatoren wie Palladium auf Aktivkohle verwendet werden. Die Reaktion kann in einem unter den Reaktionsbedingungen inerten Lösungsmittel, beispielsweise einem niederen Alkohol wie Ethanol oder einem niederen Alkylester wie Essigsäureethylester oder in Gemischen dieser Lösungsmittel durchgeführt werden. Zweckmäßig wird die katalytische Hydrierung bei einem Wasserstoffdruck von 2 bis 6 bar und bei Raumtemperatur durchgeführt.

Sofern freie Phosphonsäuregruppen und/oder freie Carbonsäuregruppen von Verbindungen der Formel I verestert werden sollen, können hierzu freie Phosphonsäuregruppen von Verbindungen der Formel I mit Verbindungen der Formel Va oder Vb auf an sich bekannte Weise umgesetzt werden. Freie Carbonsäuregruppen von Verbindungen der Formel I können mit Verbindungen der Formel Vc auf an sich bekannte Weise umgesetzt werden. Als Fluchtgruppen Y in Verbindungen der Formeln Va, Vb und Vc eignen sich beispielsweise Halogen, insbesondere Chlor oder Brom, oder Reste von Niederalkansulfonsäuren wie beispielsweise Trifluormethansulfonyloxy, oder von aromatischen Sulfonsäuren wie von Benzolsulfonsäuren, oder von durch niederes Alkyl oder Halogen substituierten Benzolsulfonsäuren wie Toluolsulfonsäuren.

Sofern Verbindungen der Formel I hergestellt werden sollen, worin R¹ und R² jeweils die gleiche, aber eine von R³ verschiedene Bedeutung besitzen, geht man zweckmäßigerweise von Ausgangsverbindungen der Formel II aus, worin R¹⁰¹ und R²⁰¹ identische Bedeutungen besitzen und von Ausgangsverbindungen der Formel III, worin R³⁰² eine von R¹⁰¹ und R²⁰¹ verschiedene Bedeutung besitzt. Beispielsweise können unter hydrogenolytischen Bedingungen stabile Phosphonsäureschutzgruppen R¹⁰¹ und R²⁰¹ gewählt werden wie niederes Alkyl, vorzugsweise Ethyl. Gleichzeitig kann als Carbonsäureschutzgruppe R³⁰² eine unter hydrogenolytischen Bedingungen abspaltbare Gruppe wie die Benzylgruppe verwendet werden. Unter den Bedingungen einer katalytischen Hydrierung wird dann in erhaltenen Verbindungen der Formel IV nur die Benzylgruppe R³⁰² zur freien Carbonsäure gespalten, während die Ethylgruppen R¹⁰¹ und R²⁰¹ erhalten bleiben. Gewünschtenfalls kann anschließend die freie Carbonsäure mit einer Verbindung der Formel Vc verestert werden. Ebenso können in Verbindungen der Formel I, worin die Phosphonsäureschutzgruppen R¹⁰¹ und R²⁰¹ unter hydrogenolytischen Bedingungen stabile Gruppen wie niedere Alkylgruppen, vorzugsweise Ethyl, bedeuten und R³⁰² für eine hydrogenolytisch abspaltbare Gruppe wie die Benzylgruppe steht, zuerst die Ethylgruppen R¹⁰¹ und R²⁰¹ unter sauren Bedingungen abgespalten werden, wobei die Benzylgruppe R³⁰² erhalten bleibt. Gewünschtenfalls können dann die freien Phosphonsäuregruppen mit Verbindungen der Formel Va oder Vb, beispielsweise mit Pivaloyloxymethylchlorid, verestert werden. Anschließend kann die unter hydrogenolytischen Bedingungen abspaltbare Benzylgruppe R³⁰² durch katalytische Reduktion mit Wasserstoff unter an sich bekannten Bedinungen abgespalten werden, um Verbindungen der Formel I zu erhalten, worin R³ Wasserstoff bedeutet.

Sofern Verbindungen der Formel I gewünscht sind, worin R¹ und R² unterschiedliche Bedeutungen besitzen, kann zweckmäßigerweise von Ausgangsverbindungen der Formel II ausgegangen werden, worin R¹⁰¹ und R²⁰¹ verschiedene Bedeutungen besitzen. Beispielsweise können Verbindungen der Formel II als Ausgangsverbindungen gewählt werden, worin R¹⁰¹ Wasserstoff bedeutet und R²⁰¹ für eine unter hydrogenolytischen Bedingungen stabile Phosphonsäure-Schutzgruppe steht. Beispielsweise kann R²⁰¹ für niederes Alkyl, vorzugsweise für Ethyl stehen. Gewünschtenfalls können die erhaltenen Verbindungen der Formel I, worin R¹⁰¹ für Wasserstoff steht, nachfolgend noch mit geeigneten Verbindungen der Formel Va umgesetzt werden, um Verbindungen der Formel I zu erhalten, worin R¹ und R² unterschiedliche biolabile Ester bildende Gruppen bedeuten. Die Ausgangsverbindungen der Formel II, worin R¹⁰¹ Wasserstoff bedeutet, können beispielsweise aus Verbindungen der Formel II, worin R¹⁰¹ für eine unter hydrogenolytischen Bedingungen abspaltbare Gruppe wie Benzyl steht, durch katalytische Hydrierung unter an sich bekannten Bedingungen erhalten werden.

Bei den vorstehend beschriebenen Umsetzungen werden die chiralen Kohlenstoffatome in den Ausgangsverbindungen der Formel III nicht verändert, so daß je nach Art der Ausgangsverbindungen isomerenreine Verbindungen der Formel I oder Isomerengemische erhalten werden können. Zur Herstellung von stereochemisch einheitlichen Verbindungen der Formel I werden zweckmäßigerweise stereochemisch einheitliche Verbindungen der Formel II mit stereochemisch einheitlichen Verbindungen der Formel III umgesetzt. Falls eine Verbindung der Formel II, welche kein chirales Phosphoratom enthält, mit einer racemischen Verbindung der Formel III umgesetzt wird, wird ein Gemisch aus zwei Enantiomeren der Verbindung der Formel I erhalten. Das Enantiomerengemisch kann gewünschtenfalls auf an sich bekannte Weise getrennt werden, beispielsweise durch chromatographische Trennung an chiralen Trennmaterialien oder durch Umsetzung einer freien Carbonsäure der Formel I mit geeigneten optisch aktiven Basen, beispielsweise (-)-α-Methylbenzylamin, und anschließende Auftrennung der optischen Antipoden durch fraktionierte Kristallisation der gewonnenen Salze.

Die Ausgangsverbindungen der Formel II können nach an sich bekannten Methoden erhalten werden.

So können Verbindungen der Formel II beispielsweise erhalten werden, indem man Verbindungen der allgemeinen Formel VI, 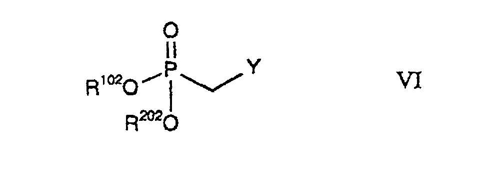 worin R¹⁰² und R²⁰² jeweils für Phosphonsäureschutzgruppen stehen und Y die obige Bedeutung besitzt, mit Cyclopentancarbonsäure der Formel VII 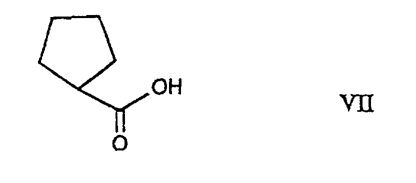 umsetzt und gewünschtenfalls anschließend die Schutzgruppen R¹⁰² und/oder R²⁰² auf an sich bekannte Weise wieder abspaltet. Beispielsweise können Verbindungen der Formel VI eingesetzt werden, worin Y für den Rest einer Niederalkansulfonsäure, vorzugsweise für Trifluormethansulfonyloxy, steht.

Die Reaktion kann auf an sich bekannte Weise unter den Bedingungen einer nucleophilen Substitution in einem unter den Reaktionsbedingungen inerten organischen Lösungsmittel durch Reaktion der Cyclopentancarbonsäure mit einer starken, zur Bildung des Dianions der Cyclopentancarbonsäure befähigten Base und anschließender Umsetzung mit dem Phosphonsäureesterderivat der Formel VI erfolgen. Als Lösungsmittel eignen sich beispielsweise offenkettige Dialkylether wie Diethylether oder cyclische Ether wie THF. Als starke Basen eignen sich beispielsweise nicht-nucleophile organische Alkalimetallamide wie Lithiumdiisopropylamid (= LDA). Zweckmäßig wird die Cyclopentancarbonsäure in THF mit zwei Äquivalenten LDA umgesetzt und das Reaktionsgemisch wird anschließend mit einer Verbindung der Formel VI weiter umgesetzt. Die Reaktionstemperatur kann zwischen -70° und 0 °C betragen.

Verbindungen der Formel VI können auf an sich bekannte Weise erhalten werden, beispielsweise indem man die Phosphonsäurediester der allgemeinen Formel VIII 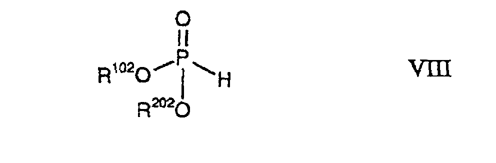 worin R¹⁰² und R²⁰² obige Bedeutungen besitzen, mit einer Quelle für Formaldehyd, beispielsweise mit Paraformaldehyd, umsetzt. Zweckmäßig kann die Reaktion lösungsmittelfrei und unter Beteiligung von in dem Reaktionsgemisch löslichen Basen durchgeführt werden. Als Basen können die oben für die Umsetzung von Verbindungen der Formel II mit Verbindungen der Formel III beschriebenen nicht-nucleophilen Basen verwendet werden. Zweckmäßig kann die Reaktion bei Temperaturen zwischen 50° und 130 °C, vorzugsweise zwischen 80° und 120 °C ausgeführt werden. Gewünschtenfalls können erhaltene Verbindungen der Formel VI, worin Y Hydroxy bedeutet, anschließend auf an sich bekannte Weise in Verbindungen der Formel VI überführt werden, worin Y für eine abspaltbare Fluchtgruppe steht.

Verbindungen der Formel VIII sind bekannt oder können nach bekannten Verfahren hergestellt werden. So können beispielsweise mit zwei unterschiedlichen biolabilen Gruppen veresterte Phosphonsäurederivate der Formel VIII hergestellt werden, indem man aus den Phosphonsäurediestern der allgemeinen Formel VIII, worin R¹⁰¹ und R²⁰¹ jeweils für die gleiche Gruppe, beispielsweise für niederes Alkyl, stehen, unter Einwirkung einer Base wie einem Alkalimetallhydroxid, beispielsweise Natriumhydroxid, zuerst eine der beiden Estergruppen abspaltet und den erhaltenen Monoester oder dessen Salz anschließend mit einer entsprechenden Verbindung der Formel Va oder Vb umsetzt. Zur Beschleunigung der Reaktion können geeignete Katalysatoren wie Tetraniederalkylammoniumsalze, beispielsweise Tetrabutylammoniumhydroxid, zugesetzt werden. Zweckmäßigerweise können dem Reaktionsgemisch zusätzlich geeignete Alkalimetallhalogenide wie Alkalimetalliodide, beispielsweise Natriumiodid zugesetzt werden, um den Reaktionsablauf zu beschleunigen. Die Reaktion kann in einem dipolaraprotischen Lösungsmittel wie einem niederen Alkylcyanid, beispielsweise Acetonitril, einem niederen aliphatischen Ether wie Diethylether, THF oder Dioxan, in Dimethylformamid (= DMF), in Dimethylsulfoxid (= DMSO) oder in Gemischen dieser Lösungsmittel durchgeführt werden. Geeignete Temperaturen hierfür liegen zwischen 0 °C und 80 °C, vorzugsweise zwischen 5 °C und 40 °C.

Verbindungen der Formel III sind aus der europäischen Patentanmeldung, Veröffentlichungsnummer 0 733 642, bekannt und können nach den dort beschriebenen Methoden hergestellt werden.

Die Verbindungen der Formel I und ihre pharmakologisch akzeptablen Salze zeichnen sich durch interessante pharmakologische Eigenschaften aus. Insbesondere hemmen die Substanzen das Endothelin Convertierende Enzym (ECE) und die Neutrale Endopeptidase (NEP) und besitzen somit ein zur Behandlung von Herzinsuffizienz besonders günstiges Wirkprofil.

Bei Herzinsuffizienz kommt es durch eine krankheitsbedingte reduzierte Auswurfleistung des Herzens zu einem reflektorisch erhöhten peripheren Gefäßwiderstand. Dadurch muß der Herzmuskel gegen eine erhöhte Nachlast anpumpen. Dies führt in einem Teufelskreis zu erhöhter Anstrengung für das Herz und verschlechtert die Situation weiter. Die Erhöhung des peripheren Widerstandes wird unter anderem durch das vasoaktive Peptid Endothelin vermittelt. Endothelin ist die stärkste derzeit bekannte körpereigne vasokonstriktorische Substanz und entsteht aus der Vorstufe Big-Endothelin unter Mitwirkung des Enzyms ECE.

Beim Krankheitsbild der Herzinsuffizienz kommt es durch die verminderte kardiale Auswurfleistung und die Erhöhung des peripheren Widerstandes zu Rückstauphänomenen des Blutes in den Lungenkreislauf und das Herz selbst. Dadurch kommt es zu einer erhöhten Wandspannung des Herzmuskels im Bereich der Vorhöfe und Kammern. In einer solchen Situation funktioniert das Herz wie ein endokrines Organ und sezerniert unter anderem das Peptid ANP (Atriales Natriuretisches Peptid) in die Blutbahn. Durch seine ausgeprägt vasodilatorische und natriuretisch/diuretische Aktivität bewirkt ANP sowohl eine Reduktion des peripheren Widerstandes als auch eine Verminderung des zirkulierenden Blutvolumens. Die Konsequenz ist eine ausgeprägte Vor- und Nachlastsenkung. Dies stellt einen endogenen kardioprotektiven Mechanismus dar. Dieser positive endogene Mechanismus ist dadurch limitiert, daß ANP nur eine sehr kurze Halbwertzeit im Plasma hat. Grund dafür ist, daß das Hormon sehr schnell durch die NEP abgebaut wird.

Die erfindungsgemäßen Verbindungen verhindern durch eine Hemmung der ECE-Aktivität die Entstehung von Endothelin und wirken so einer Erhöhung des peripheren Widerstandes entgegen, was in der Konsequenz zu einer Entlastung des Herzmuskels führt. Die erfindungsgemäßen Substanzen führen ferner durch eine Hemmung der NEP-Aktivität zu höheren ANP-Spiegeln und einer verlängerten Wirkdauer von ANP. Dies führt zu einer Verstärkung des ANP-vermittelten endogenen kardioprotektiven Wirkmechanismus. Insbesondere weisen die Substanzen eine hohe Effektivität bezüglich einer Verstärkung der diuretisch/ natriuretischen ANP-induzierten Aktivitäten auf.

NEP ist nicht nur an dem Abbau von ANP sondern auch am Abbau von Endothelin beteiligt. Daraus folgt, daß eine reine NEP-Inhibition neben der erwünschten Erhöhung der ANP-Spiegel auch zu einer ungünstigen Erhöhung der Endothelinspiegel führen würde. Aus diesem Grunde ist ein gemischtes Profil aus ECE- und NEP-Inhibition als besonders günstig anzusehen, da es sowohl den Abbau des natriuretisch/diuretisch wirkenden ANPs verhindert (NEP-Blockade), als auch gleichzeitig die Entstehung von Endothelin inhibiert (ECE-Hemmung). Dadurch kommt der negative Begleiteffekt reiner NEP-Inhibitoren (Erhöhung der Endothelinspiegel) nicht mehr zum tragen.

### 1. Bestimmung der minimalen toxischen Dosis

Gruppen von jeweils 10 Ratten von 250 g Körpergewicht (Alter 5 bis 6 Wochen) werden i.v.-Maximaldosen von 215 mg/kg der Testsubstanzen (gelost in 0,1 n wäßriger NaOH-Lösung, welche durch Zugabe von HCl auf einen pH von 7,1 eingestellt worden war) verabreicht. Die Tiere werden ab dem Zeitpunkt der Applikation 5 Stunden lang sorgfältig auf klinisch zu erkennende Anzeichen von Toxizität beobachtet. Darüberhinaus werden sie 2 mal täglich bis zum Ablauf von einer Woche beobachtet. Nach Ablauf dieser Woche wird jedes einzelne Tier vollständig seziert und alle Organe makroskopisch untersucht. Wenn Tod oder starke toxische Symptome beobachtet werden, werden weiteren Ratten zunehmend geringere Dosen verabreicht, bis keine toxischen Symptome mehr auftreten. Die niedrigste Dosis, welche Tod oder starke toxische Symptome hervorruft, wird als minimale toxische Dosis bestimmt. Die Testsubstanz des Herstellungsbeispiels 2 zeigte in der Dosis von 215 mg/kg i. v. keine signifikanten Anzeichen von Toxizität.

### 2. In vitro Untersuchung der NEP-Hemmwirkung der Substanzen

Zum Nachweis für die Hemmwirkung der erfindungsgemäßen Substanzen auf die Neutrale Endopeptidase (= NEP) wurde in einem Standardtest in vitro die inhibitorische Wirkung der Substanzen auf den durch die enzymatische Aktivität der NEP stattfindenden hydrolytischen Abbau von Methionin-Enkephalin (= Met-Enkephalin) untersucht. Dabei wurde als Maß für die inhibitorische Wirksamkeit der Substanzen deren IC₅₀-Wert bestimmt. Der IC₅₀-Wert einer enzyminhibierend wirksamen Testsubstanz ist diejenige Konzentration der Testsubstanz, bei der 50 % der enzymatischen Aktivität des NEP blockiert wird.

### Testdurchführung

Zur Testdurchführung werden jeweils 100 µl Proben verschiedener Inkubationslösungen enthaltend 10 ng gereinigte NEP (E.C. 3.4.24.11) und jeweils unterschiedliche Mengen an Testsubstanz und 20 µM Substrat (Met-Enkephalin) und 50 mM Trispuffer (= Tris(hydroxymethyl)aminomethan/HCl, pH 7,4) hergestellt.

Pro Testsubstanz werden 6 verschiedene Inkubationslösungen hergestellt mit 3 verschiedenen Testsubstanzkonzentrationen jeweils als Doppelbestimmungen.

Bei jedem Test werden jeweils auch zweierlei Kontroll-Inkubationslösungen mitbehandelt, zum einen Enzymkontrollen, welche keine Testsubstanz enthalten, und zum anderen Substratkontrollen, welche weder Enzym noch Testsubstanz enthalten.

Die Inkubationslösungen werden für 30 Minuten bei 37 °C im Schüttel-Wasserbad inkubiert. Dabei wird die Enzymreaktion nach 15 Minuten durch Zugabe des Substrates (Met-Enkephalin) gestartet und am Ende der Inkubationszeit durch Erhitzen für 5 Minuten bei 95 °C gestoppt. Anschließend wird die gestoppte Inkubationslösung für 3 Minuten bei 12.000 x g zentrifugiert, und in dem Überstand werden die Konzentrationen an nicht umgesetztem Substrat und an durch die enzymatische Reaktion gebildeten Hydrolyseprodukten bestimmt. Hierzu werden jeweils Proben der Überstände durch Hochdruckflüssigchromatographie (= HPLC) an hydrophobisiertem Kieselgel aufgetrennt, und die Produkte der enzymatischen Reaktion und nicht umgesetztes Substrat werden photometrisch bei einer Wellenlänge von 205 nm bestimmt. Für die HPLC-Trennung wird eine Trennsäule (4,6 x 125 mm), welche als Reversed-Phase-Trennmaterial Nucleosil® C 18, 5 µ beinhaltet, eingesetzt. Der Lösungsmittelfluß beträgt 1,0 ml/min, die Säule wird auf 40 °C erwärmt. Fließmittel A ist 5 mM H₃PO₄, pH 2,5 und Fließmittel B ist Acetonitril + 1 % 5 mM H₃PO₄, pH 2,5.

Aus den in den verschiedenen Proben gemessenen Konzentrationen an Hydrolyseprodukten und nicht umgesetztem Substrat wird für die Teststubstanzen der IC₅₀-Wert auf an sich bekannte Weise berechnet. Die Testsubstanz des Herstellungsbeispiels 2 zeigte in diesem Test einen IC₅₀-Wert für NEP-Hemmung von 1,7 nM und erwies sich somit als hochpotenter NEP-Inhibitor.

### 3. In vivo Bestimmung des Einfluß der Substanzen auf Diurese/Natriurese in volumenbelasteten Ratten

Die in vivo Aktivität wurde in der volumenbelasteten Ratte untersucht. In diesem Experiment wird durch eine Infusion von isotonischer Natriumchloridlösung ein hoher kardialer Füllungsdruck verursacht, in dessen Folge es zu einer ANP-Freisetzung und dadurch zu einer Diurese/Natriurese kommt.

### Testdurchführung

Die Versuche werden mit männlichen Wistar Ratten mit einem Körpergewicht von 200 - 400 g durchgeführt. Unter Neurolept-Analgesie (Fentanyl; Hypnorm®, Hersteller Fa. Janssen) wird ein Katheter in die rechte Femoralvene eingebunden für die Hintergrundinfusion und die Volumenbelastung mit isotonischer Natriumchloridlösung. Nach Öffnung der Bauchhöhle wird ein zweiter Katheter in die Blase eingesetzt und die Harnröhre abgebunden, so daß eine Messung von Harnvolumen, Natriurese und Kaliurese möglich wird.

Der Bauchraum wird wieder verschlossen und die Tiere bekommen eine Dauerinfusion mit Natriumchloridlösung (0,5 ml/ 100 g Körpergewicht) über den gesamten Versuchszeitraum von 2 Stunden. Nach einer Äquilibrierungsperiode von 30 Minuten werden in einer Vorlaufphase vor der Testsubstanzgabe dreimal jeweils über einen Zeitraum von 10 Minuten Urinproben gesammelt. Diese Vorwerte (= "Predrug"-Werte) werden bestimmt um zu überprüfen, daß bei den Testtieren ein kontinuierlicher Urinfluß stattfindet.

Sodann werden die die Teststubstanzen enthaltenden Lösungen intravenös (Bolus-Injektion in die Femoralvene) oder oral (mittels Schlundsonde) an Gruppen von jeweils 10 Ratten verabreicht. Für beide Appliktionsformen erhält jeweils eine Kontrolltiergruppe lediglich Placebo-Lösungen, die keinen Wirkstoff enthalten. 5 Minuten nach i.v. Applikation bzw. 120 Minuten nach oraler Gabe der Substanzen werden die Ratten mit einem erhöhten Volumen Natriumchloridlösung i.v. belastet (2 ml/100 g Körpergewicht in 2 Minuten) und der Urin wird über einen Zeitraum von 60 Min. gesammelt. Die in diesem Zeitraum anfallenden Urinmengen werden bestimmt und die darin enthaltenen Natrium- und Kaliumgehalte gemessen. Aus den angefallenen Urinmengen wird die unter der Volumenbelastung erfolgte Steigerung der Ausscheidung gegenüber den Vorlaufwerten abgelesen.

In der nachfolgenden Tabelle 1 werden die unter Volumenbelastung nach Testsubtanzgabe aufgetretenen Steigerungen der Harnausscheidung in % bezogen auf die unter Volumenbelastung nach Placebogabe erfolgte Harnausscheidung angegeben. Ferner werden auch die unter Volumenbelastung nach Testsubstanzgabe ausgeschiedenen Mengen an Natrium und Kalium in % der unter Volumenbelastung nach Placebogabe ausgeschiedenen Natriumund Kaliummengen angegeben. Die Beispielsnummern in den Tabellen 1 und 2 beziehen sich auf die nachfolgenden Herstellungsbeispiele.

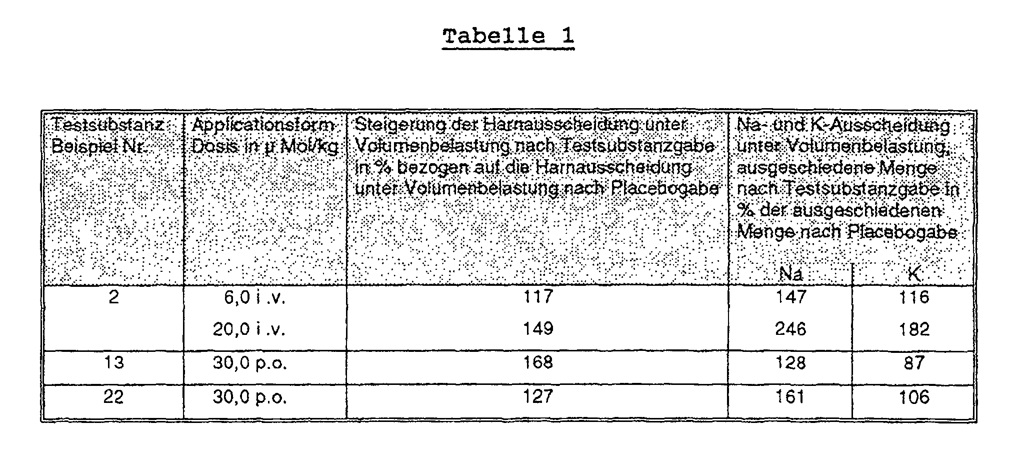

### 4. In vivo Untersuchungen der ECE-Hemmwirkung der Substanzen in Ratten

Zum Nachweis für die Hemmwirkung der erfindungsgemäßen Substanzen auf das Endothelin Convertierende Enzym (= ECE) wurde in einem Standardtest in vivo die inhibitorische Wirkung der Substanzen auf den durch die enzymatische Aktivität des ECE stattfindenden hydrolytischen Abbau von Big-Endothelin (Big-ET) zu Endothelin (= ET) untersucht. ET ist eine körpereigene stark vasokonstriktorisch wirksame Substanz. Ein Anstieg des ET-Spiegels führt zu einer Blutdrucksteigerung. Bei Infusion von Big-ET erfolgt eine Blutdrucksteigerung in dem Maße wie aus diesem durch ECE-katalysierte Spaltung ET entsteht. Als Maß für die ECE-inhibitorische Wirkung der Substanzen wurde deren Hemmwirkung auf den durch Infusion von Big-ET induzierten Blutdruckanstieg bestimmt.

### Testdurchführung

Die Versuche werden mit männlichen CD®-Ratten der Firma Charles River Wiga mit einem Körpergewicht von 220 - 280 g durchgeführt. Unter Ketamin/ Xylazin-Narkose wird den Tieren ein Katheter zur Substanzapplikation in die linke Jugularvene und ein zweiter zur Messung des Blutdruckes in die linke Arteria Carotis eingebunden. Nach 30 Minuten Erholungszeit wird den Tieren die Testsubstanz als Lösung intravenös (= i.v.) oder intraduodenal (= i.d.) verabreicht. Nach Applikation der Testsubstanzen erhalten die Tiere jeweils Big-ET in einer Dosierung von 0,5 nmol/kg intravenös. Der Zeitraum zwischen Applikation der Testsubstanz und der Gabe von Big-ET beträgt bei i.v.-Applikationen jeweils 5 Minuten, bei der i.d.-Applikation der Testsubstanzen der Beispiele Nr. 18 und Nr. 22 jeweils 15 Minuten und bei der i.d.-Applikation der Testsubstanzen der Beispiele Nr. 8 und Nr. 20 jeweils 30 Minuten. Während der nächsten 30 Minuten werden der systolische und der diastolische Blutdruck alle 5 Minuten registriert. Bei unbehandelten Tieren führt die Infusion von 0,5 nmol/kg Big-Endothelin reproduzierbar zu einer drastischen Blutdruckerhöhung, die ca. 30 Minuten anhält. Das Maximum der Blutdruckerhöhung ist nach ca. 5 Minuten erreicht.

In der nachfolgenden Tabelle 2 wird die maximale Erhöhung des Blutdruckes nach Big-ET Applikation bei mit Placebolösung behandelten Kontrolltieren und bei Tieren, die mit den Testsubstanzlösungen in verschiedenen Dosierungen vorbehandelt wurden, angegeben.

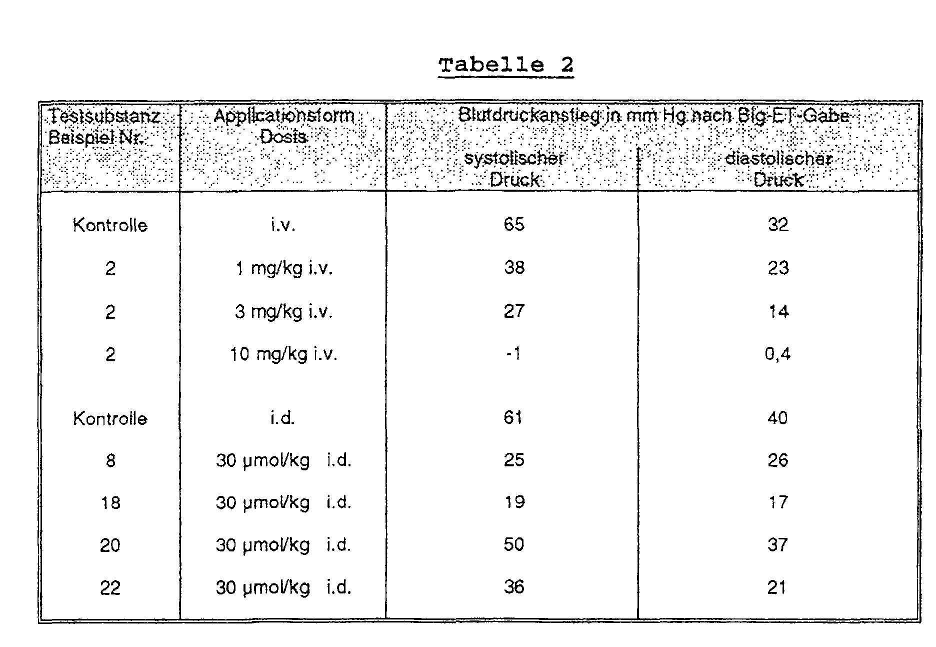

Die vorstehenden Testergebnisse zeigen, daß die Verbindungen der Formel I hohe Affinitäten zu ECE und NEP besitzen und dosisabhängig durch Hemmung der ECE-Aktivität der Entstehung von ET und einer durch dieses induzierten Erhöhung von peripherem Gefäßwiderstand und Blutdruck entgegenwirken. Die Testergebnisse zeigen auch, daß die Substanzen außerdem noch durch Hemmung des ANP-abbauenden Enzyms (NEP) zu einer Erhöhung des ANP-Spiegels im Blut beitragen und dadurch die durch ANP ausgelösten diuretisch/natriuretischen Effekte erhöhen, ohne einen wesentlichen Kaliumverlust zu verursachen.

Aufgrund ihrer vorstehend beschriebenen Wirkung sind die Verbindungen der Formel I geeignet als Arzneimittel für größere Säugetiere, insbesondere Menschen, zur Behandlung von Herzinsuffizienz und zur Förderung der Diurese/Natriurese, insbesondere bei an Herzinsuffizienz leidenden Patienten. Hierbei werden Verbindungen der Formel I und deren Salze und biolabile Ester zweckmäßig in oral applizierbaren Arzneiformen eingesetzt. Die zu verwendenden Dosen können individuell verschieden sein und variieren naturgemäß je nach Art des zu behandelnden Zustandes, der verwendeten Substanz und der Applikationsform. Im allgemeinen eignen sich jedoch für Applikationen an größeren Säugetieren, insbesondere Menschen, Arzneiformen mit einem Wirkstoffgehalt von 1 bis 200 mg pro Einzeldosis.

Als Heilmittel können die Verbindungen der Formel I mit üblichen pharmazeutischen Hilfsstoffen in galenischen Zubereitungen, wie z. B. Tabletten, Kapseln, Suppositorien oder Lösungen, enthalten sein. Diese galenischen Zubereitungen können nach an sich bekannten Methoden hergestellt werden unter Verwendung üblicher fester oder flüssiger Trägerstoffe wie z.B. Milchzucker, Stärke oder Talkum oder flüssigen Paraffinen und/oder unter Verwendung von üblichen pharmazeutischen Hilfsstoffen, beispielsweise Tablettensprengmitteln, Lösungsvermittlern oder Konservierungsmitteln.

Die nachfolgenden Beispiele sollen die Erfindung näher erläutern, jedoch deren Umfang in keiner Weise beschränken.

Die Strukturen der neuen Verbindungen wurden durch spektroskopische Untersuchungen, insbesondere durch Analyse der IR-Spektren und gegebenenfalls Bestimmung der optischen Drehwerte gesichert.

### Beispiel 1:

### (3S)-3-(1-Dibenzylphosphonomethyl-cyclopentan-1-carbonylamino)-2,3,4,5-tetrahydro-2-oxo-1H-1-benzazepin-1-essigsäurebenzylester

A) Unter Rühren wurden 100 ml Dibenzylphosphit, 12,5 g Paraformaldehyd und 6,2 ml Triethylamin zusammengegeben. Bei langsamer Erwärmung auf 55 °C erfolgte ein Temperaturanstieg auf 120 °C. Die nun klare Lösung ließ man auf 90 °C abkühlen und rührte noch 30 Minuten bei dieser Temperatur. Nach Abkühlen auf Raumtemperatur wurde an 1 kg Kieselgel bei erhöhtem Druck chromatographiert (Laufmittel: n-Hexan/Essigsäureethylester 1:4). Nach Einengen der Fraktionen und 12-std. Trocknen des Rückstands im Vakuum bei 60 °C wurden 96,1 g reines, öliges Dibenzylhydroxymethylphosphonat erhalten, welches ohne weitere Reinigung umgesetzt wurde.
B) 17,8 g Dibenzylhydroxymethylphosphonat wurden in 120 ml trockenem Dichlormethan gelöst. Nach Abkühlen auf -50 °C wurden unter Feuchtigkeitsausschluß nacheinander zunächst 7,3 g 2,6-Lutidin, dann 10,6 ml Trifluormethansulfonsäureanhydrid zugetropft. Das Reaktionsgemisch wurde erst eine Stunde lang bei -50 °C gerührt, danach noch 1 Stunde bei 0 °C. Zur Aufarbeitung wurde dieses Gemisch auf eiskaltes Wasser gegossen und die organische Phase erst mit verdünnter eiskalter Salzsäure, dann mit eiskaltem Wasser gewaschen. Nach Trocknung der organischen Phase über Natriumsulfat und Filtration wurde im Vakuum eingedampft. Das erhaltene Rohprodukt wurde an 200 g Kieselgel chromatographiert (Laufmittel: n-Hexan/Essigsäureethylester 3:2). Nach Einengen und Trocknen der Produktfraktionen wurden 17,0 g öliges Dibenzylphosphonomethyltrifluormethylsulfonat erhalten.
C) Unter Stickstoffatmosphäre und Feuchtigkeitsausschluß wurden 16,5 ml Diisopropylamin in 100 ml trockenem THF gelöst und auf -70 °C abgekühlt. Zu diesem Gemisch wurden 65,5 ml einer 1,6-molaren Lösung von n-Butyllithium in n-Hexan zugetropft. Dann ließ man 30 Minuten bei 0 °C rühren, kühlte auf -20 °C ab und ließ bei dieser Temperatur eine Lösung von 5,3 ml Cyclopentancarbonsäure in 20 ml THF zutropfen. Dieses Reaktionsgemisch wurde erst 30 Minuten bei -20 °C, dann 2 Stunden bei 0 °C gerührt und anschließend auf -60 °C abgekühlt. Zu diesem Gemisch wurde eine Lösung von 20,0 g des oben unter B) erhaltenen Produktes in 20 ml THF langsam zugetropft. Nach beendigter Zugabe wurde 1 Stunde lang bei -30 °C und dann eine Stunde bei -20 °C gerührt. Dann wurde das Reaktionsgemisch auf eiskalte wäßrige Kaliumhydrogensulfatlösung gegossen und mit Methyl-tert.butylether (= MTBE) extrahiert. Die organische Phase wurde abgetrennt, mit gesättigter Kochsalzlösung gewaschen, über Natriumsulfat getrocknet und nach Filtration im Vakuum eingeengt. Das erhaltene Rohprodukt wurde durch Chromatographie an 300 g Kieselgel mit reinem MTBE, dem ein von 5 % auf 10 % kontinuierlich steigender Methanolanteil zugemischt wurde, gereinigt. Das so erhaltene Produkt wurde zur weiteren Reinigung erneut an 200 g Kieselgel chromatographiert, wodurch 6,7 g reine 1-Dibenzylphosphonomethyl-1-cyclopentancarbonsäure erhalten wurde, Fp.= 89 - 92°C.
D) Zu einer auf 65 °C erhitzten Lösung von 24,5 g racemischem 3-Amino-2,3,4,5-tetrahydro-2-oxo-1H-1-benzazepin-1-essigsäure-tert.-butylester in 54 ml Ethanol wurde eine Lösung von 12,65 g L-(+)-Weinsäure in 54 ml auf 65 °C erhitztem Ethanol gegeben. Das Reaktionsgemisch wurde eine Stunde bei Raumtemperatur gerührt. Dann wurde eine Lösung von 1,72 ml Benzaldehyd in 1,3 ml Ethanol hinzugetropft. Die erhaltene Suspension wurde 14 Stunden bei 80 °C am Rückfluß gekocht und dann auf Raumtemperatur abgekühlt. Der entstandene kristalline Niederschlag wurde abgesaugt, in 80 ml Ethanol aufgenommen und erneut 8 Stunden am Rückfluß gekocht. Dann wurde auf Raumtemperatur abgekühlt und die Kristalle wurden abgesaugt und unter vermindertem Druck bei 50 °C getrocknet. Es wurden 23,6 g weinsaures Salz mit einem Schmelzpunkt von 195 bis 196 °C; [α]²⁰_{D} = - 152,0 ° (c = 0,5 in Methanol) erhalten.
E) Zur Freisetzung der Base wurden 23,6 g des weinsauren Salzes in einem Gemisch aus 250 ml Wasser und 108 ml Dichlormethan unter Rühren auf 0 °C abgekühlt und durch Zusatz von wäßriger Ammoniak-Lösung auf pH 9,6 eingestellt. Die organische Phase wurde abgetrennt, die wäßrige Phase nochmals mit 30 ml Dichlormethan extrahiert und die organischen Phasen vereinigt, über Natriumsulfat getrocknet und unter vermindertem Druck eingeengt. Der verbleibende Rückstand wurde aus MTBE kristallisiert und unter vermindertem Druck getrocknet. Es wurden 12,2 g (3S)-3-Amino-2,3,4,5-tetrahydro-2-oxo-1H-benzazepin-1-essigsäure-tert.-butylester, Fp.= 113° - 115 °C; [α]²⁰_{D} = -276,2 ° (c = 0,5 in Methanol) erhalten.
F) 3,6 g des vorstehend erhaltenen enantiomerenreinen tert.-Butylesters wurden mit 2,8 g p-Toluolsulfonsäure und 6,9 ml Benzylalkohol in 60 ml Toluol zusammengegeben. Anschließend wurde dieses Gemisch 3 Stunden am Wasserabscheider gekocht, das Toluol im Vakuum abgezogen und der verbleibende Rückstand mit MTBE gerührt. Nach Abdekantieren des Lösungsmittels wurde der Rückstand in Dichlormethan aufgenommen und mit eiskalter, verdünnter wäßriger Natriumcarbonat-Lösung geschüttelt. Die wäßrige Phase wurde mit Dichlormethan extrahiert und die vereinigten organischen Phasen wurden mit Wasser gewaschen. Anschließend wurde die organische Phase über Natriumsulfat getrocknet und im Vakuum eingedampft. Der Rückstand wurde aus MTBE kristallisiert und getrocknet. Man erhielt 3,2 g (3S)-3-Amino-2,3,4,5-tetrahydro-2-oxo-1H-benzazepin-1-essigsäurebenzylester, Fp. = 113 - 115 °C; [α]²⁰_{D} = -236,8° (c = 0,5 in Methanol).
G) 5,8 g der oben unter C) erhaltenen Säure wurden in 148 ml trockenem Dichlormethan aufgenommen. Der erhaltenen Lösung wurden unter Eiskühlung nacheinander 4,8 g des vorstehend erhaltenen Produktes, 3,7 ml N-Methylmorpholin, 1,84 g 1-Hydroxybenzotriazol und 5,8 g N-(3-Dimethylaminopropyl)-N'-ethylcarbodiimid-hydrochlorid hinzugefügt. Anschließend ließ man das Reaktionsgemisch unter Feuchtigkeitsausschluß 1 Stunde bei Raumtemperatur rühren. Zur Aufarbeitung wurde das Reaktionsgemisch mit Dichlormethan verdünnt und nacheinander mit Wasser, wäßriger Kaliumhydrogensulfatlösung, Wasser, wäßriger Natriumbicarbonatlösung und erneut mit Wasser gewaschen. Trocknung der organischen Phase über Natriumsulfat und Eindampfen im Vakuum lieferten 10,5 g Rohprodukt, das chromatographisch an 200 g Kieselgel (Laufmittel: n-Hexan/Essigsäureethylester 3:7) gereinigt wurde. Nach Eindampfen der Produktfraktionen und Trocknen im Vakuum wurden 6,5 g der reinen Titelverbindung als fester Schaum isoliert, IR: 3400, 3310, 2940, 1740, 1650 cm⁻¹ (Film); [α]²⁰_{D} = -104,6° (c = 0,754 in Methanol).

### Beispiel 2:

### (3S)-3-(1-Phosphonomethyl-cyclopentan-1-carbonylamino)-2,3,4,5-tetrahydro-2-oxo-1H-1-benzazepin-1-essigsäure

A) 1,9 g (3S)-3-(1-Dibenzylphosphonomethyl-cyclopentan-1carbonylamino)-2,3,4,5-tetrahydro-2-oxo-1H-1-benzazepin-1essigsäurebenzylester (Herstellung siehe Beispiel 1G) wurden in 100 ml Ethanol gelöst. Dazu gab man 1,2 g eines 5%igen Palladiumkatalysators auf Aktivkohle und hydrierte 3 Stunden lang bei einem Wasserstoffdruck von 5,5 bar. Zur Aufarbeitung wurde vom Katalysator abfiltriert, im Vakuum eingedampft und getrocknet. Man erhielt 0,9 g der Titelverbindung als schaumiges Produkt, IR: 3400, 1720, 1630 cm⁻¹ (KBr); [α]²⁰_{D} = - 140,8° (c = 0,5 in Methanol).
B) 701 mg der vorstehend erhaltenen freien Säure und 238 mg Natriumcarbonat wurden in 60 ml Wasser gelöst und die Lösung wurde im Vakuum eingedampft. Der erhaltene Rückstand wurde in wenig MTBE aufgenommen und erneut im Vakuum eingedampft. Der nunmehr erhaltene feste Schaum wurde aus Isopropanol kristallisiert, die Kristalle wurden vom Lösungsmittel abgetrennt und 2 Tage im Hochvakuum bei 60 °C getrocknet. Man erhielt 700 mg des Natriumsalzes der Titelverbindung, Fp. > 270 °C; [α]²⁰_{D} = -159,7° (c = 0,149 in Methanol).

### Beispiel 3:

### (3S)-3-(1-Benzylethylphosphonomethyl-cyclopentan-1-carbonylamino)-2,3,4,5-tetrahydro-2-oxo-1H-1-benzazepin-1-essigsäurebenzylester

A) Unter Eiskühlung ließ man eine Lösung von 8,0 g NaOH in 30 ml Wasser und 30 ml Ethanol zu 27,6 g Diethylphosphit zutropfen und rührte 2 Stunden lang bei Raumtemperatur. Anschließend wurde im Vakuum eingeengt und der wäßrige Rückstand 4 mal mit MTBE extrahiert. Eindampfen der wäßrigen Phase im Vakuum lieferte 25,0 g Natriumethylhydrogenphosphit als weißes Pulver, welches ohne weitere Reinigung umgesetzt wurde.
B) Zu einer Lösung von 33,9 g Tetrabutylammoniumhydrogensulfat in 20 ml Wasser ließ man unter Eiskühlung eine Lösung von 4,0 g NaOH in 22 ml Wasser zutropfen, wobei die Temperatur unter 25 °C gehalten wurde. Anschließend ließ man 12,5 g des oben erhaltenen Produktes, gelöst in 15 ml Wasser, bei Raumtemperatur zutropfen. Nach 15 Min. Rühren wurde von ausgefallenem Natriumsulfat abgesaugt und das Filtrat 4 mal mit je 50 ml Dichlormethan extrahiert. Die vereinigten organischen Phasen wurden über Natriumsulfat getrocknet und im Vakuum eingedampft. Der Rückstand wurde bei 40 °C 1 Stunde lang im Vakuum getrocknet, in 120 ml wasserfreiem Acetonitril gelöst und mit 7,07 ml Benzylbromid und 0,4 g Natriumjodid versetzt. Man ließ 12 Stunden lang bei 50 °C rühren, zog das Lösungsmittel im Vakuum ab und nahm den Rückstand in n-Hexan auf. Es wurde vom festen Rückstand abgesaugt, mit einem Gemisch aus n-Hexan und MTBE nachgewaschen und getrocknet. Die erhaltene Lösung wurde im Vakuum eingedampft und der Rückstand an 200 g Kieselgel chromatographiert (Laufmittel: n- Hexan/Essigsäureethylester 2:3). Man erhielt 6,7 g Benzylethylphosphit als Öl, IR: 2420, 1255, 970 cm⁻¹ (Film).
C) 18,0 g des obigen Produktes wurden mit 2,5 g Paraformaldehyd und 1,2 ml Triethylamin in der in Beispiel 1A) angegebenen Weise umgesetzt. Chromatographie an 200 g Kieselgel (Laufmittel: Essigsäureethylester) lieferte 16,5 g Benzylethylhydroxymethylphosphonat als Öl, IR: 3300, 1230, 1030 cm⁻¹ (Film) .
D) 12,0 g des vorstehend erhaltenen Produktes wurden mit 6,2 g 2,6-Lutidin und 9,0 ml Trifluormethansulfonsäureanhydrid in der in Beispiel 1B) beschriebenen Weise umgesetzt. Chromatographie an 200 g Kieselgel (Laufmittel: n-Hexan/Essigsäureethylester 2:3) lieferte 16,3 g öliges Benzylethylphosphonomethyltrifluormethylsulfonat, IR: 1410, 1245, 1210, 1010 cm⁻¹ (Film) .
E) Aus 16,08 ml Diisopropylamin, 63,8 ml 1,6-molarer n-Butyllithiumlösung in n-Hexan und 5,3 ml Cyclopentancarbonsäure wurde das Dianion der Cyclopentancarbonsäure nach der in Beispiel 1C) beschriebenen Methode hergestellt und auf die dort beschriebene Weise mit 16,0 g des oben unter D) erhaltenen Produktes umgesetzt. Chromatographie des Rohproduktes an 300 g Kieselgel (Laufmittel: zunächst n-Hexan/Essigsäureethylester 1:1, welches allmählich durch reinen Essigsäureethylester ersetzt wurde) lieferte 7,1 g reine, ölige 1-(Benzylethylphosphonomethyl)-1-cyclopentancarbonsäure, IR: 2950, 1720, 1210, 1175, 1010 cm⁻¹ (Film).
F) 3,1 g der vorstehend erhaltenen Säure wurden mit 3,2 g (3S)-3-Amino-2,3,4,5-Tetrahydro-2-oxo-1H-1-benzazepin-1essigsäurebenzylester (Herstellung siehe Beispiel 1F)), 3,3 ml N-Methylmorpholin, 1,35 g Hydroxybenzotriazol und 3,8 g N-(3-Dimethylaminopropyl)-N'-ethylcarbodiimid-hydrochlorid nach der in Beispiel 1G) beschriebenen Methode umgesetzt. Chromatographie an 200 g Kieselgel (Laufmittel: Essigsäureethylester) lieferte 2,3 g der Titelverbindung als zähes Öl, IR: 3410, 2940, 1735, 1660, 1230, 1020 cm⁻¹ (KBr); [α]²⁰_{D} = -121,6° (c = 0,495 in Methanol).

### Beispiel 4:

### (3S)-3-(1-Benzylethylphosphonomethyl-cyclopentan-1-carbonylamino)-2,3,4,5-tetrahydro-2-oxo-1H-1-benzazepin-1-essigsäureethylester

A) 5,0 g (3S)-3-Amino-2,3,4,5-tetrahydro-2-oxo-1H-benzazepin-1-essigsäure-tert.-butylester (Herstellung siehe Beispiel 1E)) und 3,75 g p-Toluolsulfonsäure wurden in 80 ml Toluol 2,5 Stunden lang am Wasserabscheider gekocht. Danach wurden portionsweise insgesamt 200 ml Ethanol hinzugegeben und das entstandene Reaktionsgemisch wurde 3,5 Stunden lang unter Rückfluß gekocht. Anschließend wurde das Gemisch im Vakuum eingeengt und der Rückstand mit Dichlormethan aufgenommen. Dann wurde mit eiskalter Natriumcarbonat-Lösung geschüttelt und die organische Phase mit Wasser neutral gewaschen. Die organische Phase wurde über Natriumsulfat getrocknet, im Vakuum eingedampft und der verbleibende Rückstand getrocknet. Man erhielt 3,6 g (3S)-3-Amino-2,3,4,5-tetrahydro-2-oxo-1H-1-benzazepin-1-essigsäureethylester, Fp. = 106,5° - 108 °C; IR = 3350, 3300, 2930, 1735, 1660 cm⁻¹ (Film); [α]²⁰D = -288,4° (c = 0,5 in Methanol).
B) 3,1 g 1-(Benzylethylphosphonomethyl)-1-cyclopentancarbonsäure (Herstellung siehe Beispiel 3E)) wurden mit 2,6 g des vorstehend erhaltenen Produktes, 3,3 ml N-Methylmorpholin, 1,35 g Hydroxybenzotriazol und 3,8 g N-(3-Dimethylaminopropyl)-N'-ethylcarbodiimid-hydrochlorid nach der in Beispiel 1G) beschriebenen Methode umgesetzt. Chromatographie an 200 g Kieselgel (Laufmittel: zunächst n-Hexan/Essigsäureethylester 1:1, welches kontinuierlich bis zur Zusammensetzung 3:7 verändert wurde) lieferte 3,7 g der Titelverbindung als Öl, IR: 3410, 2950, 1735, 1660 cm⁻¹ (Film); [α]²⁰_{D}= -113,6° (c = 0,639 in Methanol).

### Beispiel 5:

### (3S)-3-(1-Ethylphosphonomethyl-cyclopentan-1-carbonylamino)-2,3,4,5-tetrahydro-2-oxo-1H-1-benzazepin-1-essigsäureethylester

3,2 g (3S)-3-(1-Benzylethylphosphonomethyl-cyclopentan-1-carbonylamino)-2,3,4,5-tetrahydro-2-oxo-1H-1-benzazepin-1-essigsäureethylester (Herstellung siehe Beispiel 4) wurden mit 1,0 g 5%igem Palladiumkatalysator auf Aktivkohle versetzt und bei einem Wasserstoffdruck von 2,2 bar nach der in Beispiel 2 beschriebenen Methode hydriert. Die Aufarbeitung lieferte 2,4 g der Titelverbindung als Schaumharz, IR: 3400, 2950, 1740, 1650 cm⁻¹ (KBr); [α]²⁰_{D} = -162,0 ° (c = 0,324 in Methanol).

### Beispiel 6:

### (3S)-3-[1-(Pivaloyloxymethyl-ethylphosphonomethyl)-cyclopentan-1-carbonylamino]-2,3,4,5-tetrahydro-2-oxo-1H-benzazepin-1-essigsäureethylester

0,6 g (3S)-3-(1-Ethylphosphonomethyl-cyclopentan-1-carbonylamino)-2,3,4,5-tetrahydro-2-oxo-1H-1-benzazepin-1-essigsäureethylester (Herstellung siehe Beispiel 5) wurden unter Feuchtigkeitsausschluß in 20 ml DMF gelöst und anschließend mit 1,86 ml Triethylamin, 0,88 ml Pivaloyloxymethylchlorid und 0,1 g Dimethylaminopyridin versetzt. Man ließ das Reaktionsgemisch über Nacht weiterrühren, dampfte das Lösungsmittel unter vermindertem Druck ab und nahm den Rückstand in Dichlormethan auf. Die organische Phase wurde mit Wasser gewaschen und anschließend über Natriumsulfat getrocknet. Einengen im Vakuum lieferte ein Rohprodukt, das zur Reinigung an 50 g Kieselgel (Laufmittel: anfangs n-Hexan/Essigsäureethylester 3:7, wobei der Esteranteil allmählich auf 100 % erhöht wurde) chromatographiert wurde. Man erhielt 188 mg der Titelverbindung als Öl, IR = 1740, 1650 cm⁻¹ (CH₂Cl₂); [α]²⁰_{D} = -124,1 ° (c = 0,228 in Methanol).

### Beispiel 7:

### (3S)-3-[1-(5-Indanylethylphosphonomethyl)-cyclopentan-1-carbonylamino]-2,3,4,5-tetrahydro-2-oxo-1H-benzazepin-1-essigsäureethylester

480 mg (3S)-3-(1-Ethylphosponomethyl-cyclopentan-l-carbonylamino)-2,3,4,5-tetrahydro-2-oxo-1H-1-benzazepin-1-essigsäureethylester (Herstellung siehe Beispiel 5) wurden in 10 ml trockenem Dichlormethan gelöst und mit 0,28 ml Triethylamin versetzt. Diese Lösung wurde auf -50 °C gekühlt, dann wurden 0,09 ml Oxalylchlorid zugegeben. Anschließend versetzte man das Reaktionsgemisch bei -50 °C mit 200 mg 5-Indanol, ließ auf 0 °C erwärmen und rührte noch 5 Stunden lang bei Raumtemperatur. Die organische Phase wurde mit Wasser gewaschen, abgetrennt, über Natriumsulfat getrocknet und bei vermindertem Druck eingedampft. Chromatographie an 80 g Kieselgel (Laufmittel: n-Hexan/Essigsäurethylester 1:1, wobei das Lösungsmittelverhältnis kontinuierlich bis 1:4 verändert wurde) und Trocknung im Hochvakuum lieferte 220 mg der Titelverbindung als zähes Harz, IR: 1740, 1655 cm⁻¹ (CH₂Cl₂); [α]²⁰_{D} = -135,1 ° (c = 0,205 in Methanol).

### Beispiel 8:

### (3S)-3-(1-Benzylethylphosphonomethyl-cyclopentan-1-carbonylamino)-2,3,4,5-tetrahydro-2-oxo-1H-1-benzazepin-1-essigsäuretert.-butylester

5,0 g 1-(Benzylethylphosphonomethyl)-1-cyclopentancarbonsäure (Herstellung siehe Beispiel 3E)) wurden mit 5,15 g (3S)-3-Amino-2,3,4,5-tetrahydro-2-oxo-1H-1-benzazepin-1-essigsäuretert.-butylester (Herstellung siehe Beispiel 1E)), 4,1 ml N-Methylmorpholin, 2,0 g Hydroxybenzotriazol und 6,3 g N-(3-Dimethylaminopropyl) -N'-ethylcarbodiimid-hydrochlorid entsprechend der in Beispiel 1G) beschriebenen Methode umgesetzt. Das erhaltene Rohprodukt wurde an 200 g Kieselgel chromatographiert (Laufmittel: zuerst n-Hexan/Essigsäureethylester 1:1, dann reiner Ester). Man erhielt 2,6 g der Titelverbindung als Schaumharz, IR = 3410, 3350, 1735, 1655 cm⁻¹; [α]²⁰_{D} = -118,1 ° (c = 0,609 in Methanol).

### Beispiel 9:

### (3S)-3-(1-Ethylphosphonomethyl-cyclopentan-1-carbonylamino)-2,3,4,5-tetrahydro-2-oxo-1H-benzazepin-1-essigsäurebenzylester

A) 3,5 g 1-(Benzylethylphosphonomethyl)-1-cyclopentancarbonsäure (Herstellung siehe Beispiel 3E)) wurden in 150 ml Ethanol gelöst und mit 1,0 g 5%igem Pd-Katalysator auf Aktivkohle versetzt. Dann wurde 4 Stunden lang bei einem Wasserstoffdruck von 2,1 bar hydriert. Man filtrierte zweimal vom Katalysator ab, dampfte im Vakuum ein und trocknete im Hochvakuum. Man erhielt 2,60 g ölige 1-Ethylphosphonomethyl-1-cyclopentancarbonsäure, welche ohne weitere Reinigung umgesetzt wurde.
B) 2,6 g des vorstehend erhaltenen Produktes wurden unter Feuchtigkeitsausschluß in 100 ml trockenem Dichlormethan gelöst und mit 3,5 g Carbonyldiimidazol und 3,56 g (3S)-3-Amino-2,3,4,5-tetrahydro-2-oxo-1H-1-benzazepin-1-essigsäurebenzylester (Herstellung siehe Beispiel 1F)) versetzt und über Nacht gerührt. Anschließend wurde auf eine gesättigte wäßrige Kaliumhydrogensulfatlösung gegossen, die organische Phase mit Wasser neutral gewschen und über Natriumsulfat getrocknet. Das erhaltene Rohprodukt wurde an 150 g Kieselgel chromatographiert (Laufmittel: zuerst Essigsäureethylester, dem allmählich Dichlormethan bis zu einem Lösungsmittelverhältnis von 1:1 zugemischt wurde). Nach Trocknung der Produktfraktionen im Vakuum erhielt man 1,4 g der Titelverbindung als festen Schaum, IR: 3410, 1740, 1645 cm⁻¹ (KBr); [α]²⁰_{D} = -130,7 ° (c = 0,339 in Methanol).

### Beispiel 10:

### (3S)-3-(1-Diethylphosphonomethyl-1-cyclopentan-1-carbonylamino)-2,3,4,5-tetrahydro-2-oxo-1H-benzazepin-1-essigsäurebenzylester

A) 69,05 g Diethylphosphit wurden mit 14,5 g Paraformaldehyd und 6,96 ml Triethylamin analog zu dem in Beispiel 1A) beschriebenen Verfahren umgesetzt. Man erhielt 66,02 g Diethylhydroxymethylphosphonat, welches nach Trocknung im Hochvakuum ohne zusätzliche Reinigung weiter umgesetzt wurde.
B) 21,02 g des oben erhaltenen Phosphonates, 15,0 g 2,6-Lutidin und 21,8 ml Trifluormethansulfonsäureanhydrid wurden in der in Beispiel 1B) beschriebenen Weise umgesetzt. Man erhielt 32,5 g öliges Diethylphosphonomethyltrifluormethylsulfonat.
C) 30,0 g des vorstehend erhaltenen Trifluormethylsulfonates wurden mit 133 ml einer 1,6 molaren Lösung von n-Butyllithium in n-Hexan und 10,8 ml Cyclopentancarbonsäure in der in Beispiel 1C) beschriebenen Weise umgesetzt. Man erhielt 11,1 g Diethylphosphonomethyl-1-cyclopentancarbonsäure, IR: 2970, 1730, 1240, 1030 cm⁻¹ (Film).
D) 5,74 g des oben erhaltenen Carbonsäurederivates wurden mit 7,05 g (3S)-3-Amino-2,3,4,5-tetrahydro-2-oxo-1H-1-benzazepin-1-essigsäurebenzylester (Herstellung siehe Beispiel 1F)) nach der in Beispiel 1G) beschriebenen Methode umgesetzt. Das erhaltene Rohprodukt wurde durch Chromatographie an Kieselgel (Laufmittel: Essigsäureethylester) gereinigt. Man erhielt 7,95 g der Titelverbindung, IR = 3400, 1745, 1650 cm⁻¹ (Film); [α]²⁰_{D} = -130,3° (c = 0,538 in Methanol).

### Beispiel 11:

### (3S)-3-(1-Diethylphosphonomethyl-cyclopentan-1-carbonylamino)-2,3,4,5-tetrahydro-2-oxo-1H-1-benzazepin-1-essigsäure

5,3 g (3S)-3-(1-Diethylphosphonomethyl-1-cyclopentan-1-carbonylamino)-2,3,4,5-tetrahydro-2-oxo-1H-benzazepin-1-essigsäure-benzylester (Herstellung siehe Beispiel 10) wurden in 250 ml Ethanol gelöst, mit 1,5 g 5%igem Pd-Katalysator auf Aktivkohle versetzt und nach der in Beispiel 2 beschriebenen Methode hydriert. Man erhielt 4,3 g der Titelverbindung, IR = 3390, 1730, 1650 cm⁻¹ (KBr); [α]²⁰_{D} = -156,6 ° (c = 0,514 in Methanol).

### Beispiel 12:

### (3S)-3-(1-Diethylphosphonomethyl-cyclopentan-1-carbonylamino)-2,3,4,5-tetrahydro-2-oxo-1H-1-benzazepin-1-essigsäureethylester

2,34 g (3S)-3-1-Diethylphosphonomethyl-cyclopentan-1-carbonylamino-2,3,4,5-tetrahydro-2-oxo-1H-1-benzazepin-1-essigsäure (Herstellung siehe Beispiel 11) wurden unter Feuchtigkeitsausschluß in Dichlormethan gelöst, mit 1,6 ml N-Methylmorpholin, 0,63 g 1-Hydroxybenzotriazol, 2,0 g N-(3-Dimethylaminopropyl)-N'-ethylcarbodiimid-hydrochlorid und 0,6 ml Ethanol versetzt und 4 Stunden lang bei Raumtemperatur gerührt. Anschließend wurde das Reaktionsgemisch der Reihe nach mit Wasser, Kaliumhydrogensulfatlösung, Wasser, Natriumhydrogencarbonatlösung und wieder mit Wasser gewaschen. Die organische Phase wurde dann abgetrennt, über Natriumsulfat getrocknet und im Vakuum eingedampft. Das entstandene Produkt wurde an 200 g Kieselgel chromatographiert (Laufmittel: anfangs Essigsäureethylester, später ergänzt durch Zumischung von 5 % Methanol), die Produktfraktionen konzentriert und im Vakuum getrocknet. Man erhielt 1,6 g der Titelverbindung, IR = 3410, 1740, 1650, 1200, 1030 cm⁻¹ (Film); [α]²⁰_{D} = -126,1 ° (c = 0,584 in Methanol).

### Beispiel 13:

### (3S)-3-(1-Phosphonomethyl-cyclopentan-1-carbonylamino)-2,3,4,5-tetrahydro-2-oxo-1H-1-benzazepin-1-essigsäureethylester

1,3 g (3S)-3-(1-Diethylphosphonomethyl-cyclopentan-1-carbonyl-amino)-2,3,4,5-tetrahydro-2-oxo-1H-1-Benzazepin-1essigsäureethylester (Herstellung siehe Beispiel 12) wurden unter Stickstoffatmosphäre in 13 ml trockenem Dichlormethan gelöst. Unter Eiskühlung gab man 0,5 ml Bromtrimethylsilan und 0,4 ml Triethylamin zu und ließ über Nacht rühren. Überschüssiges Lösungsmittel wurde im Vakuum abgezogen und der Rückstand 15 Minuten lang in wäßrigem Aceton gerührt. Der nach Abdampfen des Lösungsmittels verbliebene Rückstand wurde in MTBE, dem wenig Dichlormethan zugesetzt worden war, aufgenommen und mit 0,53 g (S)-(-)-α-Methylbenzylamin versetzt. Der ausgefallene Feststoff wurde einmal aus Ethanol umkristallisiert, wobei man die Titelverbindung als α-Methylbenzylammoniumsalz vom Fp. = 210 - 213 °C erhielt. IR = 2940, 1750, 1650, 1200, 1045 cm⁻¹ (KBr); [α]²⁰_{D} = -141,0 ° (c = 0,2 in Methanol).

### Beispiel 14:

### (3S)-3-(1-Phosphonomethyl-cyclopentan-1-carbonylamino)-2,3,4,5-tetrahydro-2-oxo-1H-1-benzazepin-1-essigsäurebenzylester

3,8 g (3S)-3-(1-Diethylphosphonomethyl-1-cyclopentan-1-carbonylamino)-2,3,4,5-tetrahydro-2-oxo-1H-benzazepin-1-essigsäure-benzylester (Herstellung siehe Beispiel 10) wurden in 10 ml Dichlormethan gelöst, unter Eiskühlung mit 10,3 ml Trifluoressigsäure versetzt und anschließend 18 Stunden lang bei Raumtemperatur gerührt. Das Lösungsmittel wurde im Vakuum abgezogen und der verbleibende Rückstand mehrmals mit Toluol aufgenommen und jeweils erneut eingedampft. Das erhaltene Rohprodukt wurde in Dichlormethan gelöst und 3 mal mit Wasser gewaschen, dann die organische Phase abgetrennt, über Natriumsulfat getrocknet und das Lösungsmittel im Vakuum abgedampft. Trocknen im Hochvakuum lieferte 3,0 g der Titelverbindung als Öl, IR = 3400, 2950, 1745, 1640 cm-1 (KBr), [α]²⁰_{D} = - 146,5 ° (c = 0,2 in Methanol).

### Beispiel 15:

### (3S)-3-(1-Diisopropylphosphonomethyl-cyclopentan-1-carbonylamino)-2,3,4,5-tetrahydro-2-oxo-1H-1-benzazepin-1-essigsäurebenzylester

A) 50,0 g Diisopropylphosphit, 8,5 g Paraformaldehyd und 4,0 ml Triethylamin wurden nach der in Beispiel 1A) angegebenen Methode umgesetzt. Chromatographie des Rohproduktes an Kieselgel (Laufmittel: n-Hexan/Essigsäureethylester 1:4) lieferte 37,5 g Diisopropylhydroxymethylphosphonat als Öl, welches ohne Reinigung weiter umgesetzt wurde.
B) 19,6 g der vorstehend erhaltenen Verbindung wurden mit 17,4 ml Trifluormethansulfonsäureanhydrid und 11,96 g 2,6-Lutidin in der in Beispiel 1B) beschriebenen Weise umgesetzt. Chromatographie des Rohproduktes an Kieselgel (Laufmittel: n-Hexan/Essigsäureethylester 3:7) lieferte 27,4 g Diisopropylphosphonomethyltrifluormethylsulfonat als Öl, IR = 2980, 1410, 1205, 1000 cm⁻¹ (Film) .
C) 27,4 g der vorstehend erhaltenen Verbindung, 10,05 ml Cyclopentancarbonsäure und 120 ml einer 1,6 molaren Lösung von n-Butyllithium in n-Hexan wurden nach der in Beispiel 1C) beschriebenen Methode umgesetzt. Chromatographie des Rohproduktes an Kieselgel (Laufmittel: zuerst n-Hexan/ Essigsäureethylester 3:7, welchem nach und nach steigende Anteile an Ester bis 100 % zugemischt wurde) lieferte 10,6 g Diisopropylphosphonomethyl-1-cyclopentancarbonsäure vom Fp. = 53 - 57 °C.
D) 2,05 g der vorstehend erhaltenen Verbindung wurden mit 2,24 g (3S)-3-Amino-2,3,4,5-tetrahydro-2-oxo-1H-1-benzazepin-1-essigsäurebenzylester (Herstellung siehe Beispiel 1F)) nach der in Beispiel 1G) beschriebenen Methode umgesetzt. Man erhielt 3,5 g der Titelverbindung als Öl, IR = 3410, 1735, 1650, 1240, 1180 cm⁻¹ (Film); [α]²⁰_{D} = - 127,5 ° (c = 0,287 in Methanol).

### Beispiel 16:

### (3S)-3-(1-Benzylisopropylphosphonomethyl-cyclopentan-1-carbonylamino)-2,3,4,5-tetrahydro-2-oxo-1H-1-benzazepin-1-essigsäureethylester

A) 92,0 ml Diisopropylphosphit und 22,2 g NaOH wurden auf die in Beispiel 3A) beschriebene Weise umgesetzt. Man erhielt 88,0 g Natriumisopropylhydrogenphosphit, welches ohne weitere Reinigung umgesetzt wurde.
B) 88,0 g der vorstehend erhaltenen Verbindung und 34 ml Benzylbromid wurden analog zu der in Beispiel 3B) beschriebenen Methode umgesetzt. Man erhielt 46,3 g Benzylisopropylphosphit als Öl, welches ohne weitere Reinigung umgesetzt wurde.
C) 46,3 g der vorstehend erhaltenen Verbindung wurden mit 6,1 g Paraformaldehyd und 2,87 ml Triethylamin nach der in Beispiel 1A) beschriebenen Methode umgesetzt. Man erhielt 24,0 g Benzylisopropylhydroxymethylphosphonat als Öl, IR = 3300, 1230, 995 cm⁻¹ (Film).
D) 24,0 g der vorstehend erhaltenen Verbindung wurden mit 18,01 ml Trifluormethansulfonsäureanhydrid und 13,57 ml 2,6-Lutidin nach der in Beispiel 1B) beschriebenen Methode umgesetzt. Man erhielt 32,5 g Benzylisopropyl-phosphonomethyltrifluormethylsulfonat als Öl, IR = 2980, 1410, 1245, 1000 cm⁻¹ (Film).
E) 32,5 g der vorstehend erhaltenen Verbindung, 9,65 ml Cyclopentancarbonsäure und 13,4 ml einer 1,6 molaren Lösung von n-Butyllithium in n-Hexan wurden nach der in Beispiel 1C) beschriebenen Methode umgesetzt. Chromatographie des Rohproduktes an Kieselgel (Laufmittel: zuerst n-Hexan/Essigsäureethylester 1:1, dann reiner Ester, dann Essigsäureethylester mit 5 Vol-% Isopropanol) lieferte 7,0 g 1-Benzylisopropylphosphonomethyl-1-cyclopentancarbonsäure, welche ohne weitere Reinigung umgesetzt wurde.
F) 1,25 g der vorstehend erhaltenen Verbindung und 1,06 g (3S)-3-Amino-2,3,4,5-tetrahydro-2-oxo-1H-1-benzazepin-1-essigsäureethylester (Herstellung siehe Beispiel 4A) wurden nach der in Beispiel 1G) beschriebenen Methode umgesetzt. Man erhielt 0,68 g der Titelverbindung, IR = 2400, 1735, 1655, 1200, 985 cm⁻¹ (Film); [α]²⁰_{D} = -123,0 ° (c = 0,1 in Isopropanol).

### Beispiel 17:

### (3S)-3-(1-Ethylphosphonomethyl-cyclopentan-1-carbonylamino)-2,3,4,5-tetrahydro-2-oxo-1H-1-benzazepin-1-essigsäure-tert.butylester

2,2 g (3S)-3-(1-Benzylethylphosphonomethyl-cyclopentan-1carbonylamino)-2,3,4,5-tetrahydro-2-oxo-1H-1-benzazepin-1-essigsäure-tert.-butylester (Herstellung siehe Beispiel 8)) wurden mit 1,0 g eines 5%igen Pd-Katalysators auf Aktivkohle nach der in Beispiel 2 angegebenen Methode bei 2,5 bar Wasserstoffdruck hydriert. Man erhielt 1,7 g der Titelverbindung, IR: 3400, 1735, 1650 cm⁻¹ (Film); [α]²⁰_{D} = - 158,2° (c = 0,515 in Methanol).

### Beispiel 18:

### (3S)-3-[1-(Pivaloyloxymethyl-ethylphosphonomethyl)-cyclopentan-1-carbonylamino]-2,3,4,5-tetrahydro-2-oxo-1H-benzazepin-1-essigsäure-tert.-butylester

0,6 g (3S)-3-(1-Ethylphosphonomethyl-cyclopentan-1-carbonylamino)-2,3,4,5-tetrahydro-2-oxo-1H-1-benzazepin-1-essigsäuretert.-butylester (Herstellung siehe Beispiel 17) wurden mit 1,73 ml Triethylamin, 0,86 ml Pivaloyloxymethylchlorid und 0,1 g Dimethylaminopyridin nach der in Beispiel 6 angegebenen Methode umgesetzt. Nach Chromatographie an Kieselgel (Laufmittel: Essigsäureethylester) erhielt man 392 mg der Titelverbindung als zähes Harz, IR = 1740, 1650 cm⁻¹ (CH₂Cl₂); [α]²⁰_{D} = -122,9 ° (c = 0,257 in Methanol).

### Zu Beispiel 20

### (3S)-3-[1-Phosphonomethyl-cyclopentan-1-carbonylamino)-2,3,4,5-tetrahydro-2-oxo-1H-1-benzazepin-1-essigsäure-tert.butylester: Salzformen

A) 961 mg der obenbezeichneten freien Phosphonsäure wurden mit 212 mg Natriumcarbonat und 20 ml Wasser vermischt. Das entstandene Gemisch wurde filtriert und das erhaltene Filtrat wurde im Vakuum eingedampft. Der erhaltene Rückstand wurde aus Ethanol kristallisiert und die Kristalle wurden einen Tag lang bei 60 °C im Vakuum getrocknet. Man erhielt 750 mg Natrium-Salz der Titelverbindung, Fp. > 270 °C, [α]²⁰_{D} = -141,5 ° (c = 0,25 in Methanol).
B) 961 mg der obenbezeichneten freien Phosphonsäure wurden in 20 ml MTBE gelöst und mit 0,42 ml tert.-Butylamin versetzt. Die entstandene Lösung wurde im Vakuum eingedampft und der erhaltene Rückstand wurde in einem Gemisch aus MTBE/n-Hexan aufgenommen. Die in diesem Lösungsmittelgemisch entstandenen Kristalle wurden abgetrennt und bei 60 °C im Vakuum getrocknet. Man erhielt 950 mg AmmoniumSalz der Titelverbindung, Fp. = 215 °- 220° C; [α]²⁰_{D} = -149,8 ° (c = 0,26 in Methanol).

Nach den in vorstehenden Beispielen beschriebenen Verfahren können auch die in der nachfolgenden Tabelle 3 angegebenen Verbindungen der Formel I hergestellt werden.

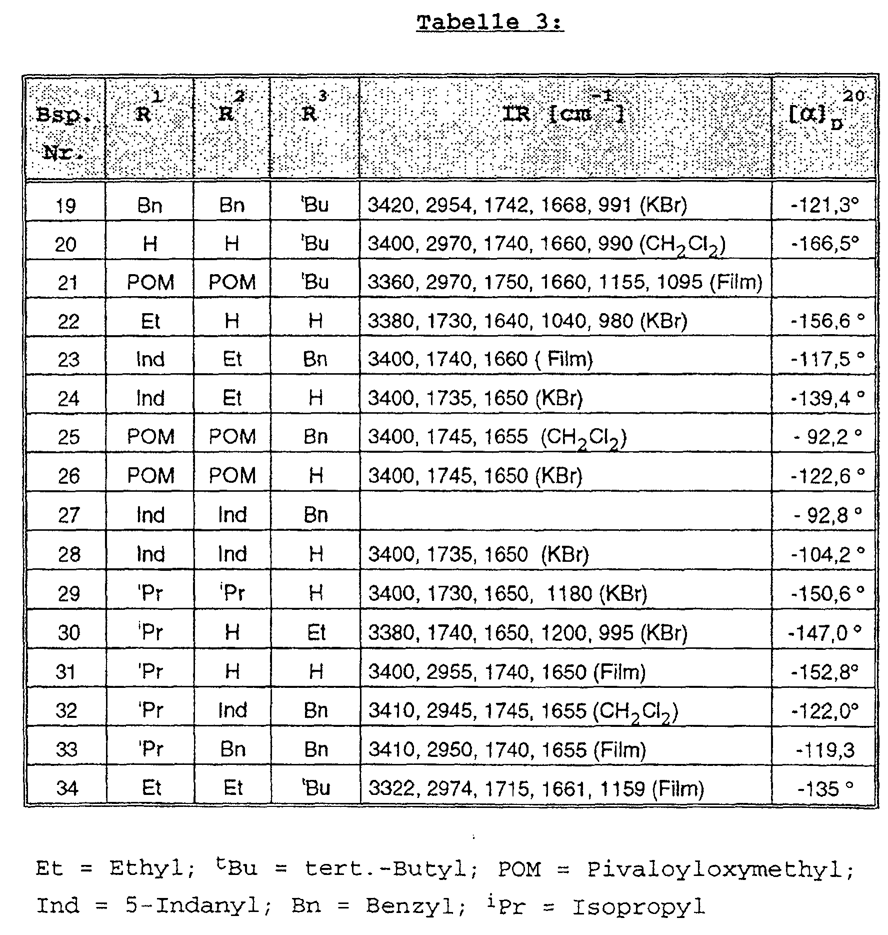

### Beispiel I:

### (3S)-3-[1-(Pivaloyloxymethyl-ethylphosphonomethyl)-cyclopentan-1-carbonylamino]-2,3,4,5-tetrahydro-2-oxo-1H-benzazepin-1-essigsäure-tert.-butylester enthaltende Kapseln:

Man stellte Kapseln in folgender Zusammensetzung pro Kapsel her:

| | |
|---|---|
| (3S)-3-[1-(Pivaloyloxymethyl-ethylphosphonomethyl)-cyclopentan-1-carbonylamino]-2,3,4,5-tetrahydro-2-oxo-1H-benzazepin-1-essigsäure-tert.-butylester | 20 mg |
| Maisstärke | 60 mg |
| Milchzucker | 301 mg |
| Essigsäureethylester | q.s. |

Der Wirkstoff, die Maisstärke und der Milchzucker wurden unter Zuhilfenahme von Essigsäureethylester zu einer homogenen pastösen Mischung verarbeitet. Die Paste wurde zerkleinert und das entstehende Granulat wurde auf ein geeignetes Blech gebracht und zur Entfernung des Lösungsmittels bei 45 °C getrocknet. Das getrocknete Granulat wurde durch eine Zerkleinerungsmaschine geleitet und in einem Mixer mit weiteren folgenden Hilfsstoffen vermischt:

| | |
|---|---|
| Talkuum | 5 mg |
| Magnesiumstearat | 5 mg |
| Maisstärke | 9 mg |

und sodann in 400 mg fassende Kapseln (= Kapselgröße 0) abgefüllt.

## Patentansprüche

1. Verbindungen der allgemeinen Formel I 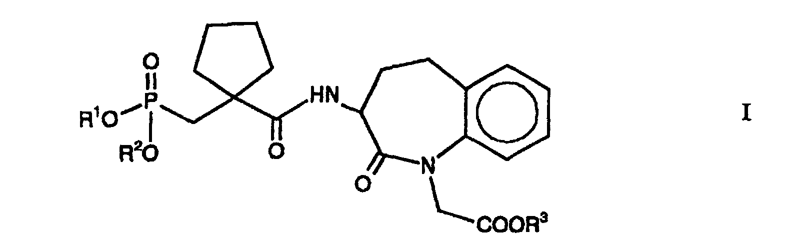 worin
R¹ Wasserstoff oder eine einen biolabilen Phosphonsäureester bildende Gruppe bedeutet,
R² Wasserstoff oder eine einen biolabilen Phosphonsäureester bildende Gruppe bedeutet, und
R³ Wasserstoff oder eine einen biolabilen Carbonsäureester bildende Gruppe bedeutet
und physiologisch verträgliche Salze von Säuren der Formel I.

2. Verbindungen gemäß Anspruch 1, worin R³ Wasserstoff oder C₁₋₄-Alkyl bedeutet.

3. Arzneimittel, enthaltend eine pharmakologisch wirksame Menge einer Verbindung gemäß Anspruch 1 und übliche pharmazeutische Hilfs- und/oder Trägerstoffe.

4. Verfahren zur Herstellung von Verbindungen der allgemeinen Formel I 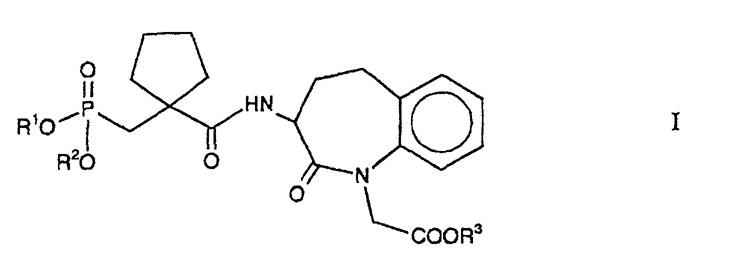 worin
R¹ Wasserstoff oder eine einen biolabilen Phosphonsäureester bildende Gruppe bedeutet,
R² Wasserstoff oder eine einen biolabilen Phosphonsäureester bildende Gruppe bedeutet, und
R³ Wasserstoff oder eine einen biolabilen Carbonsäureester bildende Gruppe bedeutet
und physiologisch verträglichen Salzen von Säuren der Formel I, **dadurch gekennzeichnet, daß** man
a) zur Herstellung von Verbindungen der allgemeinen Formel IV 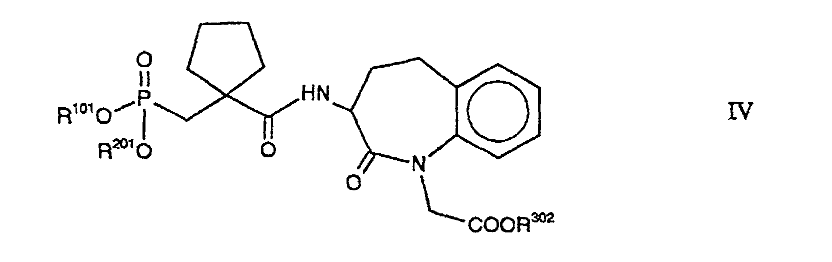 worin R¹⁰¹ und R²⁰¹ unabhängig voneinander jeweils Wasserstoff oder eine Phosphonsäureschutzgruppe bedeuten und R³⁰² eine Carbonsäureschutzgruppe bedeutet, Verbindungen der allgemeinen Formel II 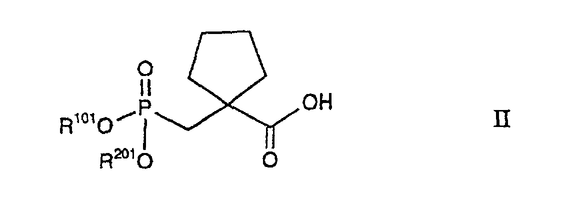 worin R¹⁰¹ und R²⁰¹ obige Bedeutungen besitzen, mit Verbindungen der allgemeinen Formel III 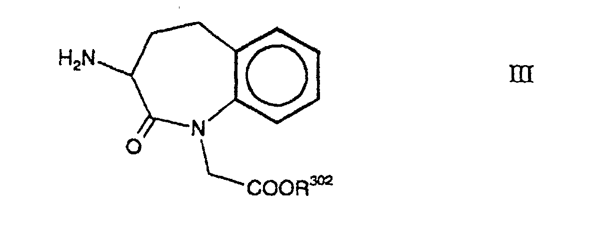 worin R³⁰² obige Bedeutung besitzt, umsetzt und, falls R¹⁰¹ und/oder R²⁰¹ Wasserstoff bedeuten, die freie(n) Phosphonsäurefunktion(en) gewünschtenfalls durch Veresterung mit einer Verbindung der allgemeinen Formel Va und/oder Vb,
R¹¹⁰-Y (Va) R²¹⁰-Y (Vb)
worin R¹¹⁰ und R²¹⁰ jeweils eine einen biolabilen Phosphonsäureester bildende Gruppe bedeuten und Y Hydroxy oder eine abspaltbare Fluchtgruppe bedeutet, in biolabile Phosphonsäureestergruppen überführt und
b) sofern in den Verbindungen der Formel IV die Schutzgruppen R¹⁰¹, R²⁰¹ und/oder R³⁰² keine gewünschten, einen biolabilen Ester bildende Gruppen darstellen, diese gleichzeitig oder einzeln in beliebiger Reihenfolge nacheinander abspaltet
und gewünschtenfalls die jeweils freiwerdenden Säurefunktionen in biolabile Estergruppen überführt, indem man freie Phosphonsäurefunktionen mit einer Verbindung der Formel Va oder Vb verestert und/oder die freie Carbonsäurefunktion mit einer Verbindung der allgemeinen Formel Vc
R³¹⁰-Y (Vc)
worin R³¹⁰ eine einen biolabilen Carbonsäureester bildende Gruppe bedeutet und Y obige Bedeutung besitzt, verestert,
und gewünschtenfalls Säuren der Formel I in ihre physiologisch verträglichen Salze überführt oder Salze der Säuren der Formel I in die freien Verbindungen überführt.

## Claims

1. Compounds of the general formula I 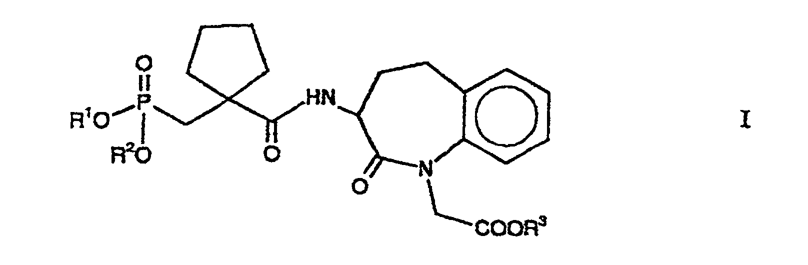 in which
R¹ is hydrogen or a group forming a biolabile phosphonic acid ester,
R² is hydrogen or a group forming a biolabile phosphonic acid ester and
R³ is hydrogen or a group forming a biolabile carboxylic acid ester
and physiologically tolerable salts of acids of the formula I.

2. Compounds according to Claim 1, in which R³ is hydrogen or C₁₋₄ alkyl.

3. Medicament, containing a pharmacologically active amount of a compound according to Claim 1 and customary pharmaceutical auxiliaries and/or excipients.

4. Process for the preparation of compounds of the general formula I 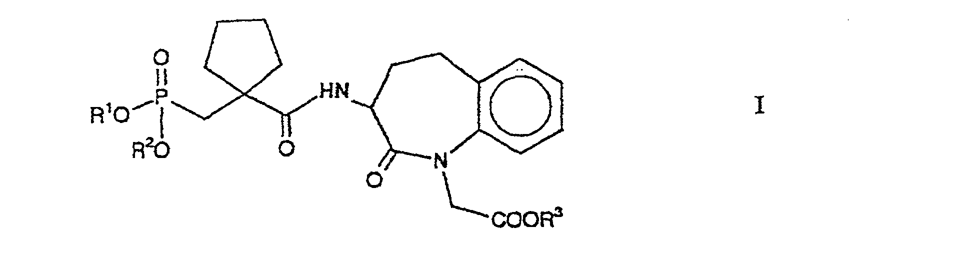 in which
R¹ is hydrogen or a group forming a biolabile phosphonic acid ester,
R² is hydrogen or a group forming a biolabile phosphonic acid ester and
R³ is hydrogen or a group forming a biolabile carboxylic acid ester
and physiologically tolerable salts of acids of the formula I, **characterized in that**
a) for the preparation of compounds of the general formula IV 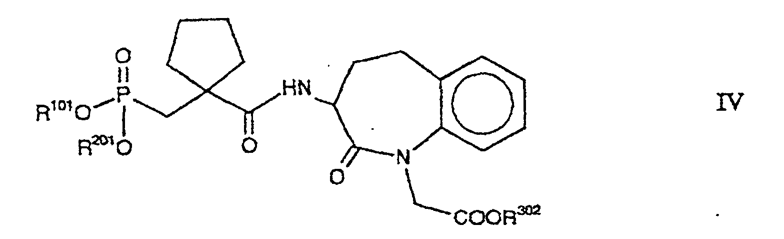 in which R¹⁰¹ and R²⁰¹ independently of one another are each hydrogen or a phosphonic acid protective group and R³⁰² is a carboxylic acid protective group, compounds of the general formula II 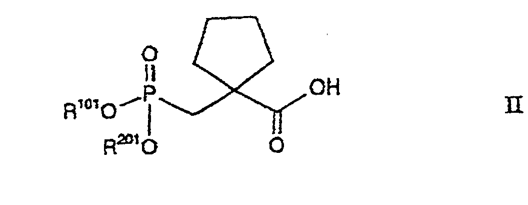 in which R¹⁰¹ and R²⁰¹ have the above meanings, are reacted with compounds of the general formula III 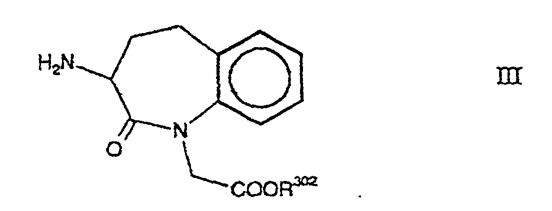 in which R³⁰² has the above meaning, and, if R¹⁰¹ and/or R²⁰¹ are hydrogen, the free phosphonic acid function(s) is/are converted, if desired, by esterification with a compound of the general formula Va and/or Vb
R¹¹⁰-Y (Va) R²¹⁰-Y (Vb)
in which R¹¹⁰ and R²¹⁰ are each a group forming a biolabile phosphonic acid ester and Y is hydroxyl or a removable leaving group, into biolabile phosphonic acid ester groups, and
b) if in the compounds of the formula IV the protective groups R¹⁰¹_{,} R²⁰¹ and/or R³⁰² are not desired groups forming a biolabile ester, these are successively removed at the same time or individually in any desired sequence
and, if desired, the acid functions liberated in each case are converted into biolabile ester groups by esterifying free phosphonic acid functions with a compound of the formula Va or Vb and/or esterifying the free carboxylic acid function with a compound of the general formula Vc
R³¹⁰-Y (Vc)
in which R³¹⁰ is a group forming a biolabile carboxylic acid ester and Y has the above meaning,
and, if desired, acids of the formula I are converted into their physiologically tolerable salts or salts of the acids of the formula I are converted into the free compounds.

## Revendications

1. Composés de formule générale I : 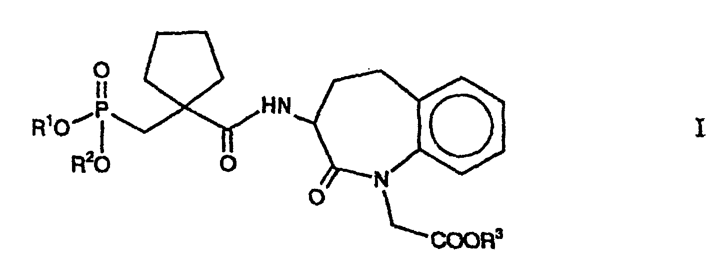 dans laquelle :
R¹ désigne l'hydrogène ou un groupe formant un ester d'acide phosphonique bio-instable,
R² désigne l'hydrogène ou un groupe formant un ester d'acide phosphorique bio-instable, et
R³ désigne l'hydrogène ou un groupe formant un ester d'acide carboxylique bio-instable,
et des sels compatibles au plan physiologique d'acides de formule I.

2. Composés selon 1, dans lesquels R³ désigne l'hydrogène ou un groupe alkyle inférieur.

3. Médicament contenant une quantité efficace au plan pharmacologique d'un composé selon la revendication 1 et des agents auxiliaires et/ou véhiculaires pharmaceutiques usuels.

4. Procédé de fabrication de composés de formule générale I : 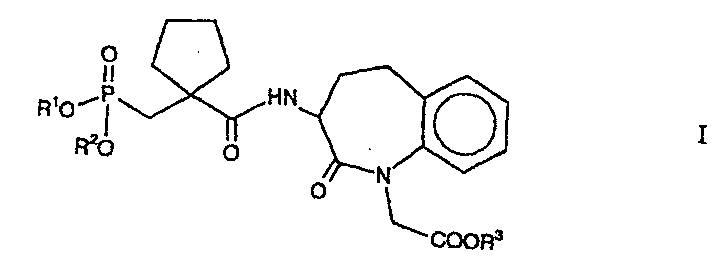 dans laquelle :
R¹ désigne l'hydrogène ou un groupe formant un ester d'acide phosphonique bio-instable,
R² désigne l'hydrogène ou un groupe formant un ester d'acide phosphonique bio-iristable, et
R³ désigne l'hydrogène ou un groupe formant un ester d'acide carboxylique bio-instable
et des sels compatibles au plan physiologique d'acides de formule I, **caractérisé en ce que** :
a) pour préparer des composés de formule générale IV : 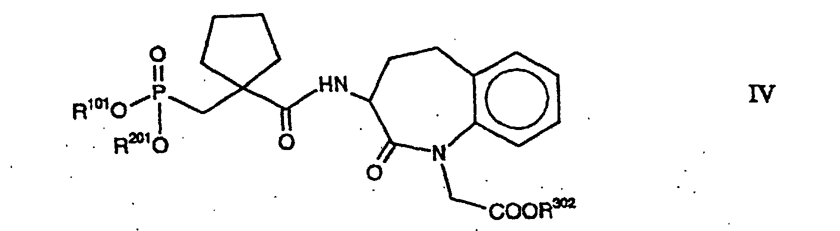 dans laquelle R¹⁰¹ et R²⁰¹ désignent respectivement indépendamment l'un l'antre de l'hydrogène ou un groupe de protection de l'acide phosphonique et R³⁰² désigne un groupe de protection de l'acide carboxylique, on fait réagir des composés de formule générale II : 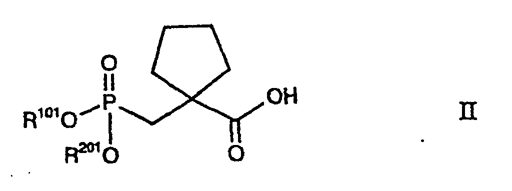 dans laquelle R¹⁰¹ et R²⁰¹ ont les significations précitées, avec des composés de formule générale III : 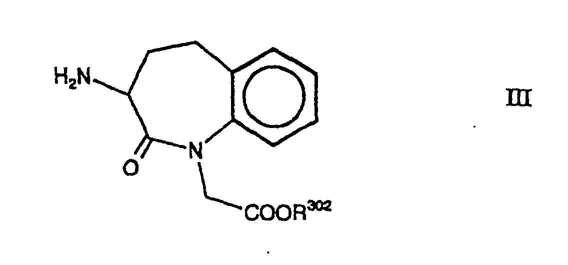 dans laquelle R³⁰² a la signification précitée et, dans le cas où R¹⁰¹ et/ou R²⁰¹ désignent l'hydrogène, on transforme la ou les fonctions acide phosphonique libres, si on le souhaite, par estérification avec un composé de formules générales Va et/ou Vb :
R¹¹⁰-Y (Va) R²¹⁰ (Vb)
dans lesquelles R¹¹⁰ et R²¹⁰ désignent respectivement un groupe formant un ester d'acide phosphonique bio-instable et Y désigne un groupe hydroxy ou un groupe volatil éliminable, en groupes esters d'acide phosphonique bio-instables, et
b) si, dans les composés de formule IV, les groupes de protection R¹⁰¹, R²⁰¹ et R³⁰² ne représentent pas de groupes souhaités formant un ester bio-instable, on sépare ceux-ci simultanément ou individuellement dans un ordre quelconque l'un après l'autre, et,
si on le souhaite, on convertit les fonctions acides qui deviennent respectivement libres en groupes esters bio-instables, on estérifie les fonctions d'acide phosphonique libres avec un composé de formule Va ou Vb et/ou on estérifiant la fonction d'acide carboxylique libre avec un composé de formule générale Vc :
R³¹⁰-Y (Vc)
dans laquelle R³¹⁰ désigne un groupe formant un ester d'acide carboxylique bio-instable et Y a la signification mentionnée ci-dessus, et,
si on le souhaite, on convertit les acides de formule I en leurs sels compatibles au plan physiologique ou les sels des acides de formule I en composés libres.
